# EUROPEAN PATENT SPECIFICATION

(11) **EP 3 381 425 B1**
(45) Date of publication and mention of the grant of the patent: **23.03.2022**
(21) Application number: 18157766.9
(22) Date of filing: 21.02.2018
(51) Int. Cl.: A61F 13/491

(54) **GENDER-SPECIFIC ABSORBENT ARTICLE**
GESCHLECHTSSPEZIFISCHER SAUGFÄHIGER ARTIKEL
ARTICLE ABSORBANT SPECIFIQUE AU SEXE

(30) Priority: 31.03.2017 ES 201700357
(43) Date of publication of application: 03.10.2018
(73) Proprietor: Giménez Lago, Rodrigo Alvaro, 36212 Pontevedra (ES)
(72) Inventor: Giménez Lago, Rodrigo Alvaro, 36212 Pontevedra (ES)
(74) Representative: Ungria López, Javier

(56) References cited:
- US-A1- 2006 282 055
- US-B1- 6 197 011
- US-B1- 6 817 992

## Description

### Technical field of the invention

This invention lies within the industry of manufacturing anatomical articles, such as absorbent dressings and diapers, for users of any sex and preferably for users of the male sex, and can be reusable or used just once (disposable).

### Background of the invention

The efforts made in the research and design of products for anatomical use with absorbent properties for human waste (urine, excrements) have been focused, on the one hand, on improving the absorption and retention of said waste, minimizing the possibility of leaks, and, on the other hand, improving the comfort and the fastening capability of the same to the user's body.

Currently there are two main devices for absorbing urine for people with urinary incontinence: urine drainage bags and diapers.

With regard to urine drainage bags, they consist of a closed bag connected to the user by means of a tube or catheter that goes directly to the inside of the bladder or, in the case of men, by means of a "collector" element in the form of a condom with a hole at the end to which the tube or catheter is connected and through which the fluid is collected when the user urinates, with the urine remaining inside the bag. The objective of such devices is to prevent the user from coming into contact with the urine, thereby avoiding skin problems, however, it has been shown that these devices do lead so some serious problems, such as:
- the risk of an infection in the urinary tract;
- discomfort, since the urine remains in a liquid state inside the bag and is moved around when the user moves; and
- it is more difficult for the user to move and, furthermore, their motion may even be limited since the bag and the tube are fastened with straps or hooks to the user's body or to a fastening element near the user (bed, wheelchair, etc.);
- there is a high risk of losing the fluid retained, such as in the case of a perforation of the bag or by a poor connection between the bag and the tube or the collector.

Moreover, one must consider that in the case of a male user, the collector element is usually adhered to the male sex organ by means of gels or adhesive elements that often cause irritation and skin problems. In the case of female users, the collector element is usually introduced in the urethra, causing possible infections and related problems.

With regard to diapers, they currently consist of a rectangular piece or body, which can also be in the form of underpants/panties, essentially consisting of two parts:
- an absorbent inner core, in contact with the user's body, the function of which is to collect and absorb the humidity produced by the urine and feces as much as possible; and
- an impermeable outer layer that has the function of preventing the urine and feces from filtering through the absorbent layer to the outside, thereby containing the fluids between the user's body and the absorbent layer until the diaper is removed.

The existence of an absorbent core for retaining fluids is described in patent ES2163615 T3, wherein said core made up of a permeable layer, which is in contact with the skin, and an impermeable layer, are disclosed. Moreover, described in the utility model ES1046784 U, aimed at improving the fastening of the diaper to the user's body by means of elastic straps or bands, is an absorbent core for retaining liquids and excrement, which in itself is a layer or material with said property and which is located between a permeable liquid-distributing layer and an impermeable material that isolates it from the outside. This structure is also repeated in other reusable diapers, such as in utility model ES 1043323 U, which describes a permeable inner layer with a smooth touch (due to the fact that it is in contact with the skin), an absorbent intermediate structure of a non-woven fabric and a permeable outer barrier. In conclusion, known diapers consists of an absorbent material that is always in contact with the skin and which is permeable to liquids, a material or core with the capacity to retain absorbed fluids and an impermeable outer layer that prevents the loss of fluids to the outside.

Thus, conventional diapers do not prevent the user's skin from coming into contact with waste (urine and feces) which, both individually and mixed can cause problems of discomfort, soreness, allergic reactions, skin reactions, etc., especially if said dirty diaper is not immediately removed and the user is not washed, since the absorption capacity of the materials used in the manufacturing of diapers is limited. The problem is worsened by the fact that both urine and feces contain chemical and biological elements that are harmful to the skin and can even interact with the composition of the diaper, leading to more harmful effects due to the mixture of all of these elements.

In light of the previous considerations with regard to the current state of the technical field of absorbent dressings and disposable diapers, the present invention proposes the development of a new absorbent product for anatomical use, preferably for a male, although it may be applied to either sex, which can be reusable or disposable and which aims to overcome the shortcomings detected, and furthermore to providing a product that is easy to use and which prevents complications and discomfort (irritation, allergies, infections) due to the humidity produced by the fluids in contact with the skin, in addition to reinforcing the retention of the same and preventing leakage.

### General description of the invention

The present invention relates to an anatomical article with absorbent and retaining properties for excremental/waste substances of the human body (urine, feces), such as a diaper or dressing, which consists of a 2. single flexible laminar body that comprises:
- a first layer, made of an impermeable material,
- a second layer, made of an absorbent material, adhered to the first layer on one of the two faces thereof and which partially or entirely covers the surface of said face,
said laminar body being configured to fold over itself and delimit a closed inner space, in the form of a container, such that the impermeable layer constitutes the outer wall in contact with the user's skin and the absorbent layer constitutes the inner wall of the space in all of, or part of, the inner perimeter thereof; and
- a hole that passes through both layers and which, in a position of use, comes into contact with the urinary organ at the impermeable outer layer, such that it allows the fluids to enter the container when the laminar body is folded over itself.

Thus, the anatomical article is intended both for female users and male 3a Documents US 6,817,992 B1 and US 6,197,011 B1 disclose disposable male incontinence diapers and garment having a housing chamber and a hole intended to allow for the urinary organ to be introduced therein, said chamber being formed by two pieces, a front wall sealably joined to a back wall. users. In the first case, the female urinary organ is in contact with (resting against) the hole on the outer layer, outside of the container, but allowing the waste to be retained within the same in a position of use (when the laminar body is folded). In the second, particular case of male users, the urinary organ is introduced in the container through the hole, in a position of use. Thus, in a particular embodiment for the male urinary organ, the anatomical article with absorbent and retaining properties for excremental/waste substances of the human body (urine, feces), such as a diaper or dressing, consists of a single flexible laminar body that comprises:
- a first layer, made of an impermeable material,
- a second layer, made of an absorbent material, adhered to the first layer on one of the two faces thereof and which partially or entirely covers the surface of said face,
said laminar body being configured to fold over itself and delimit a closed inner space, in the form of a container, such that the impermeable layer constitutes the outer wall in contact with the user's skin and the absorbent layer constitutes the inner wall of the space in all of, or part of, the inner perimeter thereof; and
- a hole that passes through both layers, intended to allow for the urinary organ to be introduced from the impermeable outer layer to the absorbent inner layer, such that it remains inside the container when the laminar body is folded over itself.

The position of the hole in the laminar body changes based on whether the anatomical article is intended for the male or female urinary organ, due to anatomical reasons which condition the position of use in each case.

Preferably, the anatomical article described is disposable, although according to certain embodiments envisaged herein, it can also be reusable or partially disposable.

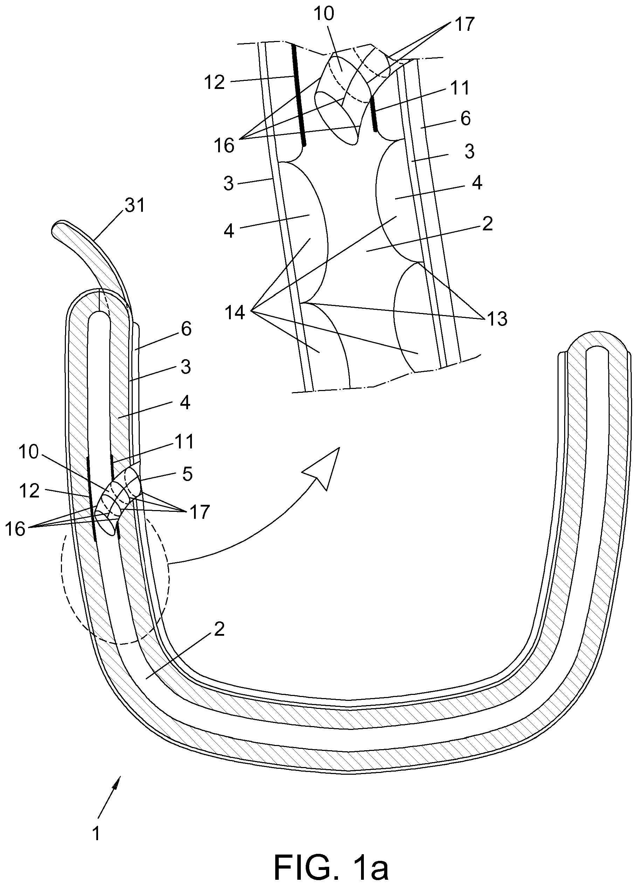

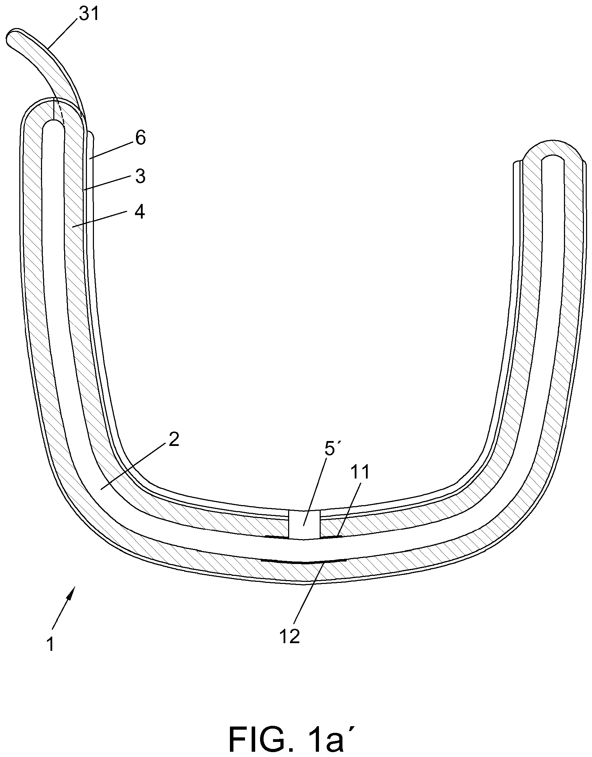

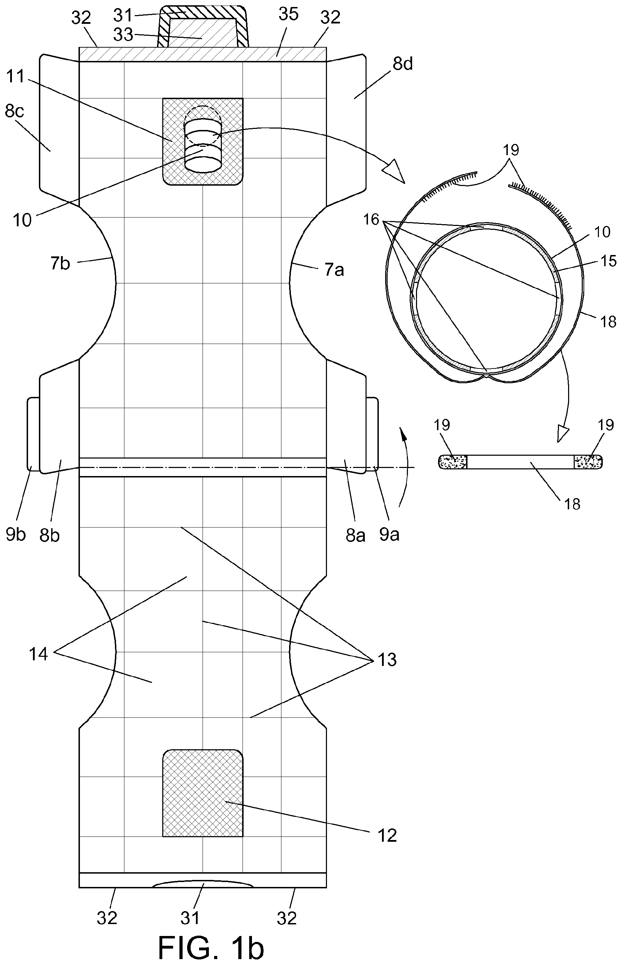

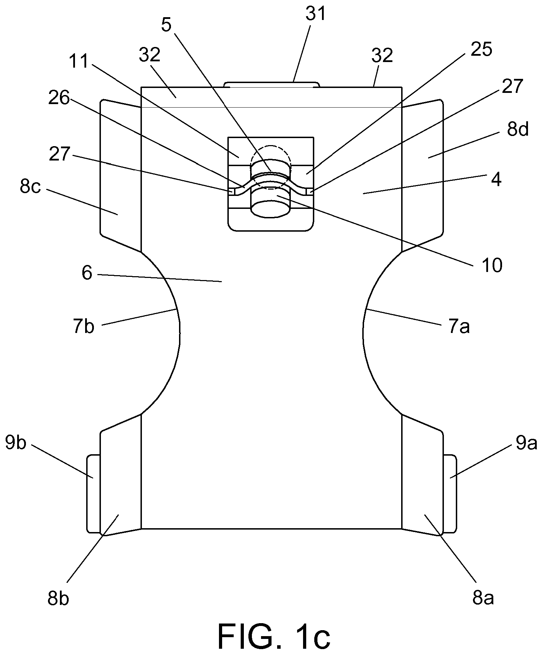

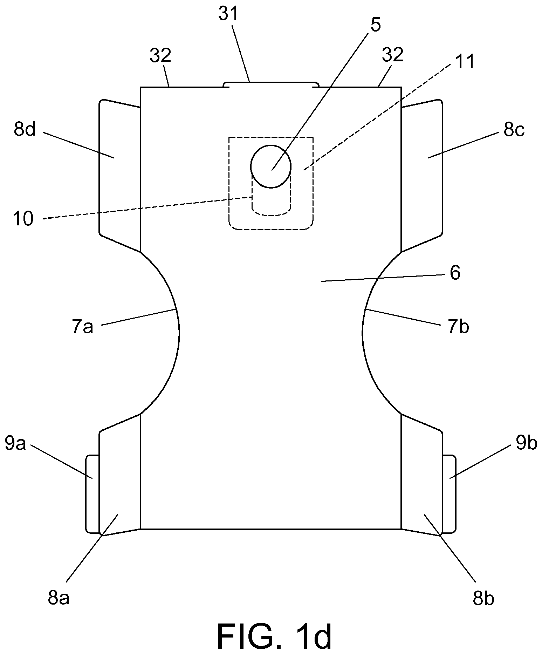

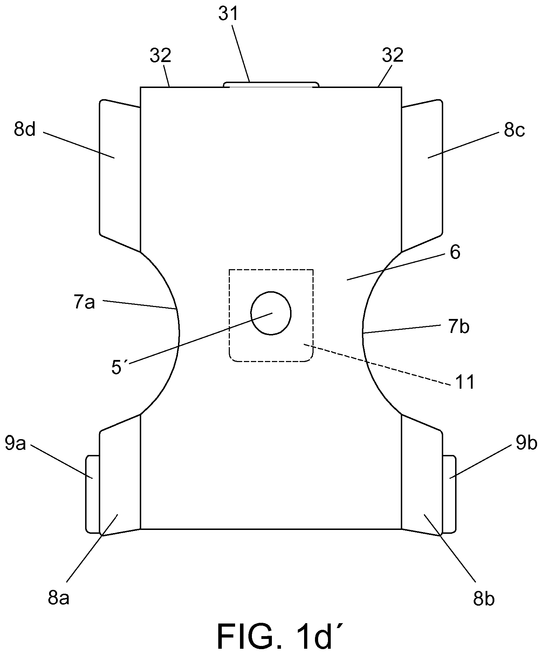

The fact that the flexible laminar body is configured to fold over itself must be understood as the ability of one end of the layers that make up said body to fold until joining with the opposite end, closing in such a way that forms a core on the inside thereof, which is a space or sealed (or semi-sealed) container, in which the urine is housed. Thus, once the laminar body is folded, it forms an article with two faces facing each other, an outer face (in contact with the environment, which is exposed to the air) and an inner face intended to be in contact with the user's body in a position of use (and specifically, with the urinary organ). In one particular case, the anatomical article or diaper is made up of a laminar body, the layers of which are already folded over themselves in an original state (in other words, when manufactured) and the sides are sealed in such a way that the two ends thereof are joined forming the container on the inside thereof, being able to be placed as such on the user's body; this alternative being similar to a conventional diaper but containing on the inside thereof a closed space or container in which the urine is retained since the hole is in contact with the urinary organ. In the case of a female urinary organ, the organ rests entirely on the hole outside of the container, while in the case of the male urinary organ the same is introduced through the hole. In another alternative case, the flexible laminar body of the anatomical article is extended in its original state, in other words, it is not folded, such that at the moment in which it is to be used, the upper and lower ends thereof are joined when placed on the user's body to form the closed inner space as well as to seal it, also by the sides thereof. Figures 1.a and 2.a-2.b show both variants of this invention, respectively.

It is evident that once the laminar body is folded over itself (in its original state or in use), the sides (meaning the sides that longitudinally extend on the laminar body) must be adhered to each other or sealed (for example through heat-sealing or by means of an adhesive system or Velcro-type system) or must be closed by means of an equivalent system so that the inner space is truly isolated from the outside and so that liquids cannot spill out. Thus, said sides can optionally incorporate adjustable, elastic side bands, which can optionally be adhesive, as in conventional diapers, to improve the fastening thereof to the body.

Considering the position of use, the anatomical article has two faces when folded over itself: an inner face, arranged to come into contact with the user's body (specifically, with the urinary organ), and an outer face, in contact with the outside.

The materials described in the present specification that make up the anatomical article (impermeable layer, absorbent layer...) are like those commonly used in manufacturing conventional diapers known by anyone with ordinary knowledge in the art. Thus, the impermeable layer can be made of a material selected from a group consisting of polypropylene, polyethylene, nitric latex, vinyl, or similar. On the other hand, the absorbent layer, without constituting a limiting aspect, but indeed a preferable one, can be made up of: a surface area of a material selected from a group consisting of polypropylene (hydrophilic non-woven fabric) or similar; an intermediate area made of cellulose, for example, such as tissue, and an inner area of a material selected from a group made up of: cellulose, wood pulp, sodium polyacrylate, bamboo or hemp fibers or similar.

Due to the configuration described for the anatomical article, either folded when manufactured or folded when in use, the contact of the fluids with the user's skin is reduced as much as possible, limited to the skin of the urinary organ (which is outside of the container and in contact with the hole in the case of a female, or which is inside the sealed space where the liquids are absorbed and retained in the case of a male). This way, the negative effects of the humidity are minimized and reduced to a small portion of the skin (a preferred embodiment prevents all contact between the body and the fluid, as will be seen). It must also be mentioned that the article prevents the movement of the fluids within the diaper, which are retained in the sealed inner space and absorbed by the absorbent layer that covers and delimits said space, eliminating the possibility of fluid loss in the case of a perforation of the layers on any part thereof (or back flow through the inlet hole) and heightening the sensation of comfort.

Likewise, the anatomical article keeps the urine and the feces separated, since the fluid is collected in the inner container through the hole and the excrement remains outside (if the anatomical article is a diaper, when it is folded, said excrement remains on the inner face which is in contact with the body), thereby annulling the negative effects of the interaction between the two. Due to the fact that the defecation takes place less frequently than diuresis, there is more time allowed between changes of the diaper/dressing, thereby saving in material and work, enabling a user to wear the diapers during longer periods of time without negatively affecting their health and comfort, the effective duration of the diaper and the frequency with which it is changed depending only on the size and absorbent capacity given to each model.

### Detailed description of the invention

The layers that make up the laminar body of the article are adhered to one another in the indicated position, and can be joined (heat-sealed, for example) at the edges (sides) thereof in order to form a single flexible laminar body, folded or able to be folded, or can have opening and closing means that allow the sides to be closed and sealed once folded when in use.

In one particular embodiment, the first, impermeable layer and the second, absorbent layer can be adhered to one another on one of the faces thereof by separable joining means, such as Velcro, a separable adhesive, or similar. This way, it is possible to separate (and remove) the absorbent inner layer (in contact with the user), separating it from the impermeable outer layer. The purpose is to reuse the anatomical article (or either partially or completely discard it or recycle it - the entire assembly or separately), since after the use thereof it is possible to separate and extract the absorbent layer, in which the fluids accumulate, to either discard it and replace it with another, or to wash it and later adhere it to the first layer. It is also possible, regardless of how both layers are adhered, to completely discard the anatomical article. The separable joining means can be distributed in different ways on the two facing faces of the impermeable layer and the absorbent layer that are adhered, as long as they provide a suitable fastening between both layers during use. Ultimately, the second, absorbent layer, can be adhered to (or removed from) the laminar body before and after its use, allowing it to be washed and used again, or discarding it to adhere a new absorbent layer, able to be completely manipulated by the user, and providing the user with the possibility of choosing the type and size of the absorbent layer to attach to the impermeable layer that best adapts to his or her needs.

Since the impermeable layer makes up the outer wall of the laminar body, part of said layer is intended to come into contact with the user's body when the laminar body is folded (the inside of the diaper), specifically with the urinary organ. This way, in a preferred case of the invention, said impermeable layer is attached to a third, covering layer that entirely or partially covers the second face of the impermeable layer, opposite the face to which the absorbent layer attached, and which is arranged to be in contact with the user's skin in a position of use (also with the outer face of the article in contact with the environment when said third layer covers the entire face of the impermeable layer). This third layer, with a thickness from 1 to 5 millimeters, both limits included, is preferably made up of a material that has a soft and smooth touch, providing a sensation of comfort to the skin, and which is also preferably made of an absorbent material (the same as or different from the first layer) to absorb and minimize the humidity produced by the bodily excretions (perspiration or sweat), or made of a washable fabric. Thus, without constituting a limiting aspect, but indeed a preferable one, the covering layer can be a single layer or can in turn be made up of several layers, preferably having a surface made of cotton, polyester, or cotton with polyester, hemp fibers, bamboo fibers, polypropylene or similar. Moreover, the covering layer can incorporate an intermediate area made of tissue or similar and an inner area made of cellulose, wood pulp, sodium polyacrylate, or another type of material with the same properties. In the most preferred case, the covering layer can be made up of these three areas: surface, intermediate and inner.

With regard to the second layer of the anatomical article, having an absorbent quality, in the most simple of cases it can have a flat surface, although more preferably it has a surface relief. For example, it can include a system of channels, grooves or striations, in a grid-like form, which distribute the fluid within the closed space and delimit centers and cores where the absorption of the urine is concentrated. Thus, a correct and continuous circulation and distribution of the retained fluid inside the closed space is enabled when the layers are folded in a uniform way throughout the absorbent layer, which is not blocked in the case of the compaction of the container.

As is evident, the part of the laminar body that is close to the hole, either at the inner wall (absorbent) or outer wall (impermeable) of the body through which said hole passes, is a relatively conflictive area, and as such, it is necessary to avoid back flow and the accumulation of the humidity of the liquids retained so that the skin is not affected, a negative effect that is worsened when the user is lying down. To reduce these risks, in a preferred embodiment, the area around the hole can be devoid of the absorbent layer, in other words the impermeable layer is not covered by the absorbent layer in said area, with the aim of depriving that area from fluid absorption capacity and preventing it from retaining humidity, allowing it to remain as dry as possible, since it is the impermeable layer that is around the hole in the container. The same is applied to the area of the impermeable layer which, when the laminar body is folded, is on the opposite side of the closed space, meaning facing the hole: this region can also be devoid of the absorbent layer, thereby leaving an area that protects against humidity, since the impermeable layer is that which is inside the container around the hole. In another preferred embodiment, the area around the hole (and the area which is facing that area) can have the absorbent layer, but with the inner face (which is not adhered to the impermeable layer) covered with a layer made of an impermeable material, in the form of a patch, which can be the same as or different from the material that makes up the first layer.

Likewise, in the particular case of the anatomical article configured for the male urinary organ, with the object of preventing back flow of the liquids through the hole, the side (inner) of the hole where the absorbent layer is located can include a film on the edge thereof made of an impermeable circular material with an elastic band, adjustable to the male urinary organ to facilitate the coupling thereof and to prevent the gaps or spaces between the hole and the urinary organ. Alternatively, as another preferred option, also in the particular case of the anatomical article configured for the male urinary organ, the anatomical article can incorporate a cannula or tube made of an impermeable, elastic and flexible material in the hole that passes through the layers. The main objective thereof consists of covering the skin of the male urinary organ that is introduced in the hole, keeping it protected and isolated from the humidity caused by the fluids inside the sealed space or container when the layers are folded. Moreover, said tube reduces the possibility of back flow through the hole. Thus, the cannula extends from the impermeable layer (or third, covering layer, if there is one), which is the entrance area of the cannula, to the absorbent layer, in such a way that it projects through said layer (outlet of the hole). This way, it remains introduced in the cavity or sealed space when the layers are folded in order to dump the urine inside the same.

This cannula must have a variable diameter and length depending on the user for whom it is intended, being personalized according to the physical characteristics of each user. In the preferred case, the cannula has the form of a condom, yet it is open at both ends, at the base or mouth (the area where the urinary organism is introduced) and at the tip (through which the urine passes into the closed inner container). Like condoms, the cannula is preferably made of a material selected from a group consisting of latex, nitrile or similar, which is elastic, flexible and impermeable. In one embodiment, the cannula or elastic and flexible tube can be made of the same impermeable material as the first layer, while in another alternative, both elements can be made of different materials. In the same way, in one particular embodiment of the invention, the cannula and impermeable layer form part of the same single piece, a unitary laminar body/cannula element, and can be manufactured joined together, while in another case the laminar body and the cannula can be manufactured separately, as two elements that make up a kit.

This way, in an alternative of the invention, the anatomical article configured for the male urinary organ can have the cannula already incorporated when manufactured, forming a unitary body, made of either the same material as the impermeable layer or of a different material. In a different alternative, it is possible to manufacture both elements separately, the anatomical article and the cannula as separate elements, such that they can be chosen, exchanged and combined according to each specific case (regardless of whether or not they are made of the same impermeable and flexible material). Thus, in one example, it is possible to have the cannula in the shape of a condom, in order to cover the male urinary organ, introducing the male urinary organ in the same, and then introducing the organ covered with the cannula in the hole.

In a recommendable configuration, although a configuration that is not essential, the cannula is rolled up in order to facilitate the introduction of the male urinary organ, in the condom-like way, but, in the opposite direction, being unrolled from the base towards the tip and not from the tip to the base (in other words, after inserting the organ through the inlet of the cannula, said cannula extends around the urinary organ and to the other end).

It is preferable for the cannula to have a greater diameter than that of the male urinary organ, providing a degree of space to facilitate the placement thereof and ventilation through the same. For these same reasons, it is convenient and preferable for the cannula to have one or more circular elastic strips on the surface thereof (joined to the cannula when manufactured), which allow the diameter of the cannula to be adjusted to the contours of the male urinary organ without compressing the same, thereby ensuring that the organ does not involuntarily come out of the cannula and preventing the possibility of a back flow of urine through the same. In another, different alternative, which is more recommendable, it is possible to use an elongated and flat elastic band or strip around the cannula with the same function as the circular elastic strips, since they are long enough to go around the cannula. Said elastic band has a opening and closing system on the ends thereof, such as a system of adhesive strips or strips with glue, the best option being a Velcro-type system, and its function consists of going around the cannula on the outer surface thereof and properly adjusting it to the urinary organ by joining the two ends thereof using the opening-closing system, without unnecessarily compressing or choking the male urinary organ, thanks to the fact that it is easily adjustable. Said elastic band can be joined to the cannula on the outer surface thereof at at least one point, or can alternatively be an independent element of the same, to which it is not joined.

Preferably, the cannula can include a layer of absorbent material (which can be made of the same material as the second layer of the anatomical article or of a different absorbent material) on the inside thereof, which is the area that is in contact with the skin of the male urinary organ. In this particular case, it is convenient, although not an mandatory condition, for this absorbent layer inside the cannula to include longitudinal bands or grooves along the same, such that said layer is not continuous along the entire circumference thereof, but rather preferably has longitudinal bands along the axis of the cannula, and between said bands having grooves in the form of expansion joints, so as not to limit the capacity of adapting to the flexibility and elasticity of the material it is made of, thereby preventing compression and choking and related problems with regard to blood supply.

In a particular case of the invention, when the cannula is separate from the laminar body (forming, for example, a kit of two elements), the base or mouth of said cannula can comprise a flat extension of two faces perpendicular with respect to the longitudinal axis of the same, wider than the diameter thereof. Said extension can have any possible form around the mouth of the cannula (circular, square, wing-shaped or in the shape of a flap...) and acts as a stop, preventing the same from being completely introduced inside the closed space. Thus, this extension of the cannula that is wider than the diameter provides a physical barrier that does not pass through the hole to the space, but rather stays on the other side, in other words, on the outer wall of the impermeable layer which comes into contact with the user's body. To further increase the fastening of the extension of the cannula to the laminar body, and since said extension has two opposite faces, it can have adhesive means or separable joining means, for example, a Velcro-type system, on the front face which stays in contact with the impermeable layer of the article (in other words, which does not come into contact with the body), to fasten said extension to said area around the hole. Preferably, if the laminar body and the cannula are two independent elements, said adhesive front face can be covered with an anti-adherent film that must be removed for use. More preferably still, the area of the impermeable layer around the hole that is in contact with the flat extension of the mouth of the cannula also includes an adhesive (preferably with an anti-adherent protector that is removed when before use) so that the fastening of the extension to the impermeable layer is reinforced, and more preferably still, both parts, the extension of the cannula and the area of the impermeable layer around the hole, include an adhesive that doubly reinforces the fastening thereof. For safety reasons, this area which has an adhesive around the hole on the face of the impermeable material through which the cannula is introduced can incorporate, in the best possible scenario, a perimeter around the hole without an adhesive (which is the impermeable layer), for example from 1 to 10 millimeters wide, both limits included, with the aim of preventing any difficulties involving the introduction of the cannula, since it is attached to the adhesive during the placement thereof.

Preferably, the face (rear) of the extension of the cannula that comes into contact with the body (opposite that which has the adhesive), incorporates a soft absorbent layer as a covering which is smooth to the touch, such as the one defined for the third optional layer. Alternatively, when the anatomical article includes a third layer that is smooth to the touch on the impermeable wall that comes into contact with the body, it is possible that said layer includes an opening in the area around the hole, with a window-type tab, which closes when the cannula is introduced. This way, once the cannula with the extension at the mouth thereof is introduced in the hole, the window of the third layer in the area around the hole opens, and the urinary organ is then introduced through the hole of the window or opening, which is in contact with the body, and through the cannula.

Regardless of whether the anatomical article includes a cannula or not, for the purpose of preventing back flow and reducing humidity in the area close to the hole, the second layer (absorbent) of the flexible body, which covers the inside of the closed space when folded, can have on its surface a core that protrudes immediately below the hole, according to the position of use. This core consists of an increase in the width of the absorbent layer in the area situated below the hole, or consists of an element that adheres to the absorbent layer in the same area, in the form of a projection or protrusion, which can be absorbent like the layer, or made of a non-absorbent material (impermeable), preferably a plastic material, such as thermoplastic (polypropylene, polyoxide, methylene, cyclic olefin copolymer, polycarbonate, polyurethane or polystyrene, among the most important ones) or resin. This way, in the particular case of the anatomical article configured for the male urinary organ, the area of the core in the absorbent layer has a thickness that is greater with respect to the thickness of the rest of the layer, with the aim of maintaining the male urinary organ (or the cannula) resting against it, and when the individual is lying down, it is elevated with respect to the surface of the absorbent layer, thereby making it difficult for urine to flow back towards the hole. In a preferred case, the core is oval-shaped or half-oval shaped (semicylindrical), in other words, with a curved surface. In another embodiment, the core has a semicircular form at the end closest to the lower part of the hole and extends in the form of a rectangle at the other end.

In the case that the anatomical article does not include a cannula, it is optional and preferred that around the contour of the hole there are: either several cores, such as the one described for the lower part thereof, or a single core going around the entire perimeter thereof, in order to create a protective area, in the form of a barrier, which limits the possibility of a back flow of urine through said hole from any direction. When there are several cores, they can have the same form or different forms.

With the aim of making sure the male urinary organ does not move to the sides when it is resting on the aforementioned core, it is possible and advantageous, yet not obligatory, for said core to comprise a safety strap in a crosswise direction with respect to the longitudinal position of the organ or cannula on the core, which is preferably elastic and which has an adjustable opening and closing system on each one of the ends thereof, for example, an adhesive or Velcro-type system, to remain attached to both sides of the core. This way, it is possible to place the male urinary organ on the core and retain it above with the fastening band or strap, preventing it from moving to the sides beyond the band and maintaining a downward orientation thereof in a position of use. To further prevent the accumulation of humidity in the area around the hole, when the core is made of an absorbent material, its surface that is in contact with the urinary organ or, in the absence thereof, with the cannula, can optionally comprise an impermeable film for covering the same that can partially or entirely cover said surface (in other words, leaving the sides of the core uncovered, the core made of an absorbent material). This way, said core has a double function when it only partially covers the surface: preventing humidity from reaching the area of the core where the organ is supported, which is covered by an impermeable material, and absorbing the urine in the uncovered absorbent areas (sides).

Although this composition and arrangement of the elements of the article is sufficient to favor the retention of liquids in the closed inner space or container, and minimally affects the user's skin, it is also possible in a preferred particular case that the core incorporates a separating or support structure (rigid or flexible) for the male urinary organ or cannula, intended to ensure a hollowing in that region of the container and prevent the compaction thereof and the accumulation of liquid where the urinary organ is housed within the same. Said structure (rigid or flexible) can have several forms, as long as a) it projects above the surface of the absorbent layer and over the organ or cannula, making a barrier against the compaction of the face opposite the container and b) it keeps the same protected in an hollow inner area, wherein said organ or cannula is housed. In the most preferred case, the structure (rigid or flexible) or support is made of a thickening of the absorbent layer or of an impermeable material, such as a plastic material, and more specifically a thermoplastic selected from a group consisting of polypropylene, polyoxide, methylene, cyclic olefin copolymer, polycarbonate, polyurethane or polystyrene, among the most important ones; or of resin. It also preferably has a U-shaped cavity (canal-type) in which the male urinary organ or cannula rests; this support with a U-shaped cavity can be made up of two equal, mirrored separated pieces (in other words, they are not joined at their base) or one single piece with said form in the central part thereof. It is possible that this support which is above the absorbent core, or which forms part of the same when it is a thickening of the absorbent layer, has two projections at the sides of the core for fastening. It is also possible that when the article has the separating structure (rigid or flexible) incorporated on the absorbent core, the fastening band or strap can be in a crosswise position around the organ or cannula or alternatively around the structure itself, over the same, which likewise retains the organ on the inside thereof by preventing it from coming out.

As was previously explained, the closed space that is inside the anatomical article can be sealed and only open at the hole that passes through the layers and which is in contact with the female urinary organ (which remains outside), or completely isolated from the outside because the hole is sealed from the passage of liquids when in use and the male urinary organ is introduced. However, it can also be semi-sealed, since, optionally, it is possible for the anatomical article to include an opening and closing system, the function of which is, among others, to make the manipulation and correct placement of the male urinary organ or cannula possible once the article is placed on the user's body, without the fluid contained in the container being able to come out; or put in a different way, the closed space formed by the fold of the layers is semi-sealed when the body is folded over itself but is in turn accessible by the user or a third person by means of the opening and closing system. This opening and closing system is ideally located in a region of the laminar body situated above the hole that passes through the layers, according to the position of use. Thus, in a preferred embodiment, the opening and closing system is positioned at the upper end of the front face of the article according to the position of use, along the width of the layers (in other words, crosswise with respect to the length of the same). In this case, it is preferable for this system to only cover the upper central part of the layers, at the height of the waistline, and not the entire width of the same from end to end, leaving one part of the laminar body on both sides without the possibility of opening, with so that when the user is lying down on their side, the fluid retained inside does not spill over at the ends. In another preferred embodiment, the opening and closing system is not located at the upper end but rather between said end and the position of the hole, on the front face of the laminar body, according to the position of use, in such a way that the system is located on the part of the laminar body which, when folded, is facing the part of the body where the hole is located; in this case, it could be either a) an opening and closing system in the same way as described for the upper end of the diaper but placed at a lower position, at any height between the position of said hole and the upper end of the diaper in a horizontal direction, in other words, parallel to the waistline of the diaper; or b) one or two front oblique systems for manipulating the inside with one hand for each opening and closing system from the outer face (front) of the diaper. Moreover, in a third alternative, it could be c) a vertical opening and closing system, perpendicular with respect to the waistline of the diaper (which corresponds to the upper end of the diaper in a position of use), situated in the area of the laminar body facing the hole when it is folded in a position of use, in the position equivalent to that of the hole (at the same height) or moved towards a side position with respect to said hole and at the same height thereof.

Said opening and closing system can be, for example, an eyelet, or more preferably an adhesive or Velcro-type tab, such that the laminar body preferably has an elongated opening and a tab that, when closed, covers said opening, thereby sealing it. This tab can close from the inside towards the outside of the closed space or from the outside inwards, being manipulable from the outside. In the best of scenarios, this tab can incorporate an absorbent layer that covers the entire inner face thereof, and which, when closed, seals the inner area of the tab against the passage of liquid. Furthermore, on the absorbent layer the tab preferably incorporates a semicylindrical (half-oval) absorbent core or projection on the inner face thereof (said absorbent core able to have another convenient shape), which is inside the closed space and which has a greater thickness than that of the second absorbent layer of the laminar body and sufficient for joining the two facing absorbent faces of the flexible folded body through contact, providing a protective core (buffer effect) that prevents the passage of fluid in that area. In these cases, the opening of the tab system can incorporate an elastic edge in order to adhere said edge to the absorbent core and make sure there is no gap between both parts, preventing the urine from flowing back through the same. With the same objective, the absorbent core of the tab can include a groove or inlet on the edge thereof where part of the impermeable layer is introduced when closed, with the aim of having the groove or edge better seal the closure of the elastic band of the opening and prevent leakage.

In an even more preferred embodiment, when the opening and closing tab is located in the upper part of the laminar body in a position of use, the second layer (absorbent) of the flexible body that covers the inside of the closed space can incorporate an absorbent core in that part, which is crosswise to the length of the layers and equal to the width of the same, above said opening and closing system. This absorbent core, like the ones herein described, consists of an increase in the thickness of the absorbent layer, or of an absorbent element that adheres to the absorbent layer, such as a projection or protrusion and which sufficiently increases the thickness thereof in order to join the two facing faces of the absorbent layer through contact when the body is folded. In one case or another, this upper absorbent core prevents the passage of retained liquid to that area and is doubly isolated, forming a type of strap, especially when the tab also incorporates an absorbent core, since both are in contact when the tab is closed, sealing the upper area from the passage of liquid and humidity.

The anatomical article described in any one of the aforementioned variants thereof can incorporate one or more adjustable and adhesive side bands to adjust the anatomical article to the user's body, such as those in a conventional diaper. The incorporation of said band or bands depends on whether the diaper is to be used as an outer diaper or an inner diaper. Likewise, the article can have a non-linear configuration on said sides of the flexible body where the adjustable elastic bands are included, adapted to improve the adjustment thereof to the body.

By way of illustration of the invention, the text is accompanied by a series of figures that show two preferred embodiments of the anatomical article: an absorbent diaper folded (sealed type) and an absorbent foldable dressing/diaper (envelope type). These two alternatives can be disposable or reusable. Basically, the first alternative is folded when manufactured and already includes the inner container formed by folding the two layers, while the second is originally unfolded, and is only folded when it is going to be used on the user's body so as to form the closed space or container. Although it is not shown in the figures, the first and second layers can be adhered by separable joining means, such as Velcro-type or a separable adhesive, such that the second, absorbent layer which collects the fluids, can be separated and can come undone from the first layer to be removed from the laminar body, and can be discarded and replaced with another, or washed and incorporated again into the diaper. Regardless of how they are adhered the first two layers that make up the flexible laminar body, the diaper can also be completely disposable in the variants that are exemplified in the figures. In fact, in the sealed model (folded when manufactured) the anatomical article does not even have to include separable joining means between the impermeable layer and the absorbent layer, the first and the second layers not being adhered but rather simply in contact, such that the absorbent layer can be removed and introduced in the container formed by the folded body through the openings made on said laminar body by manipulating the inside.

### Figures

Figure 1.a shows a transverse cross section of the "sealed type" absorbent diaper, with the container on the inside when manufactured and a cannula to receive the urinary organ. It also shows a detailed view of the container on the inside of the diaper and the inner wall that lines it.
Figure 1.a' shows a transverse cross section of the "sealed type" absorbent diaper in figure 1.a, but which is configured for female use, wherein the hole (5') changes its position in the laminar body with respect to the hole (5) for anatomical reasons.
Figure 1.b shows a plan view of the "sealed type" absorbent diaper of figure 1.a in an open position and divided into two parts in order to show the inside: the two sides of the inner wall of the container, with an opening and closing system at the upper end. It includes a front detailed view of the cannula, with an elongated and flat elastic band that has a Velcro-type opening and closing system at the ends thereof and is joined at one point to the cannula, and a detailed view of the extended elastic band and without being attached to the cannula.
Figure 1.c shows a plan view of the "sealed type" absorbent diaper in figure 1.a in an open position and divided into two parts to show the inside: the side of the inner wall of the container where the hole is, including a cannula on the absorbent core and fastened with an elastic band that has a Velcro-type opening and closing system at the two ends thereof.
Figure 1.d shows a plan view of the inner face of the "sealed type" absorbent diaper in figure 1.a that comes into contact with the user's body.
Figure 1.d' shows a plan view of the inner face of the "sealed type" absorbent diaper in figure 1.d, but which is configured for female use, wherein the hole (5') changes its position in the laminar body with respect to the hole (5) for anatomical reasons.
Figure 2.a. shows a side view of the "envelope type" absorbent diaper with a cannula, unfolded when manufactured.

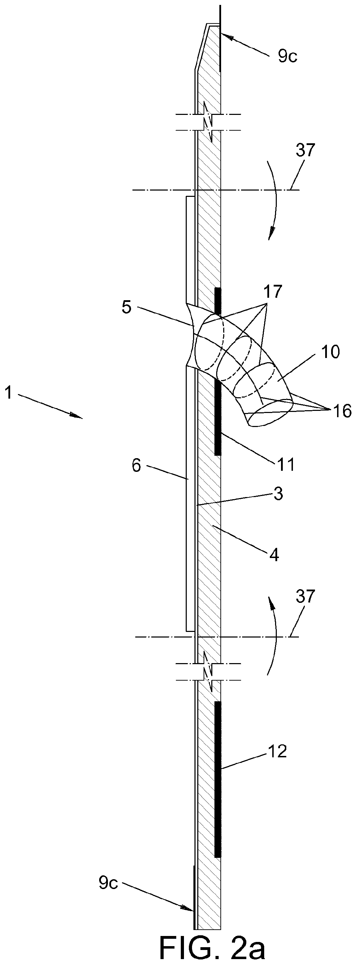

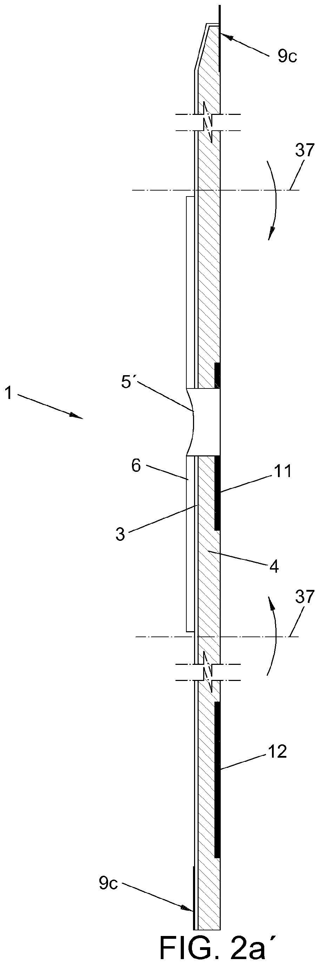

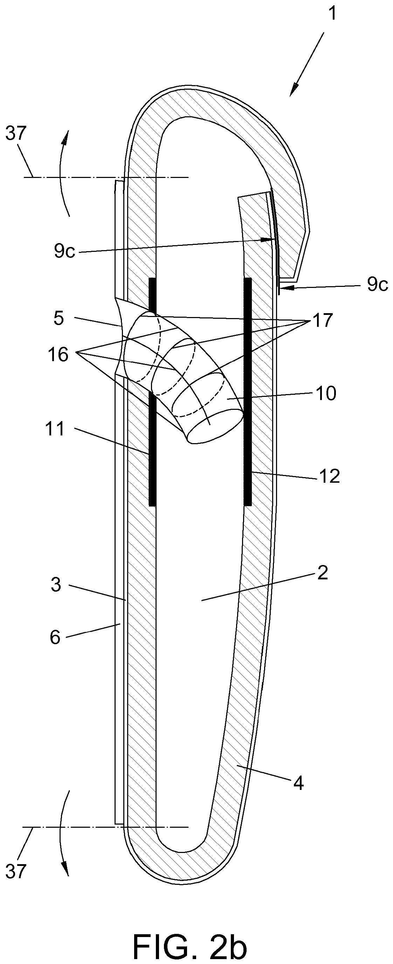

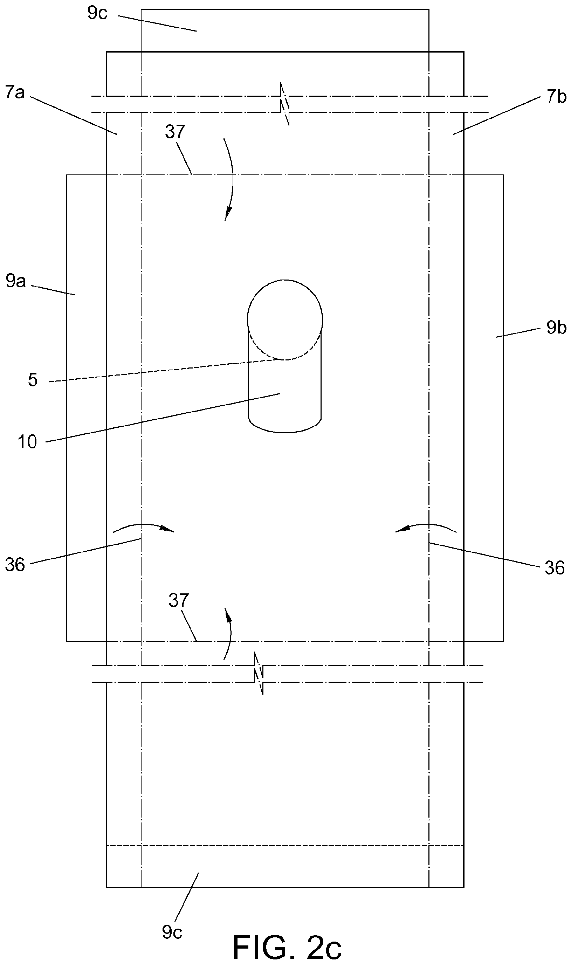

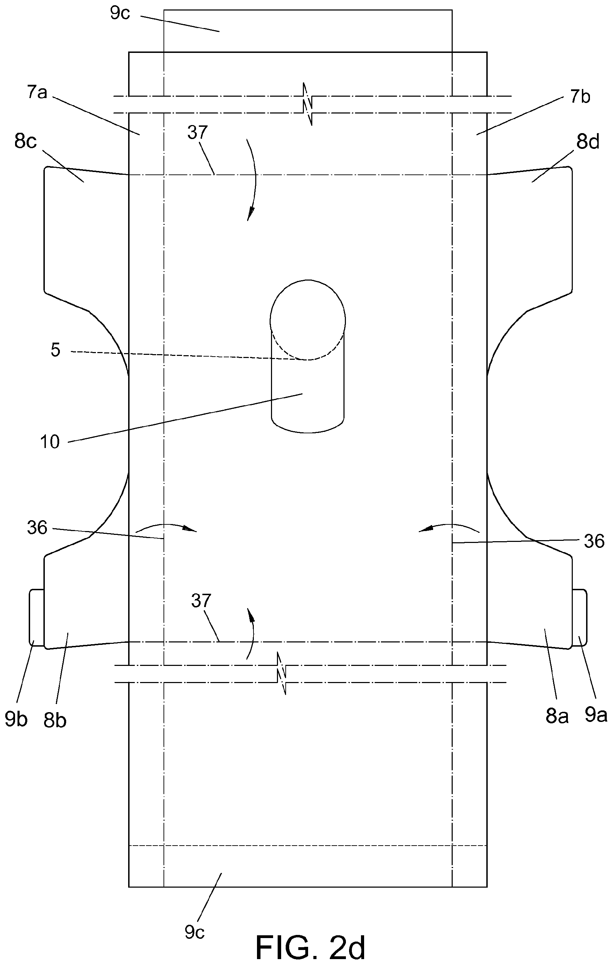

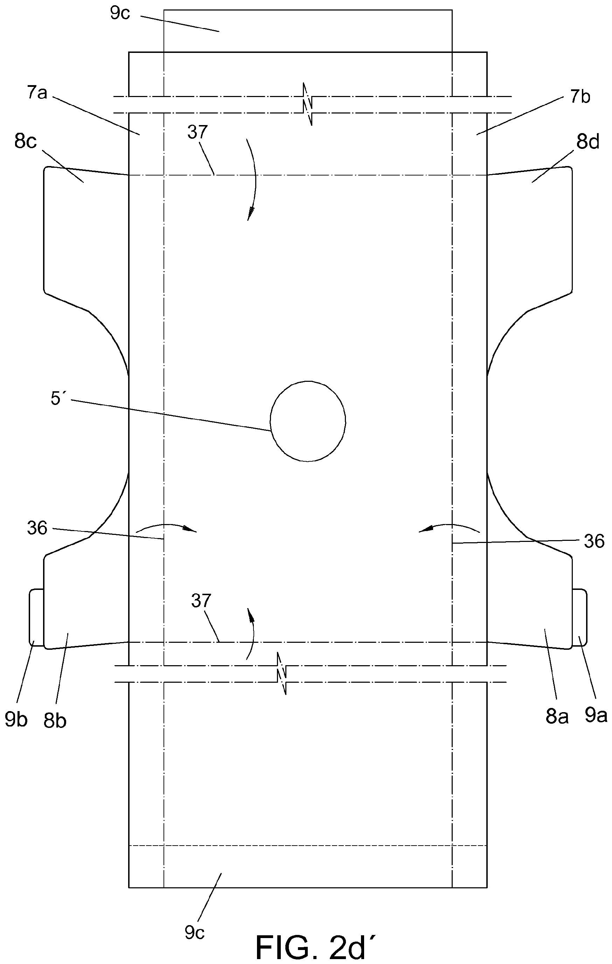

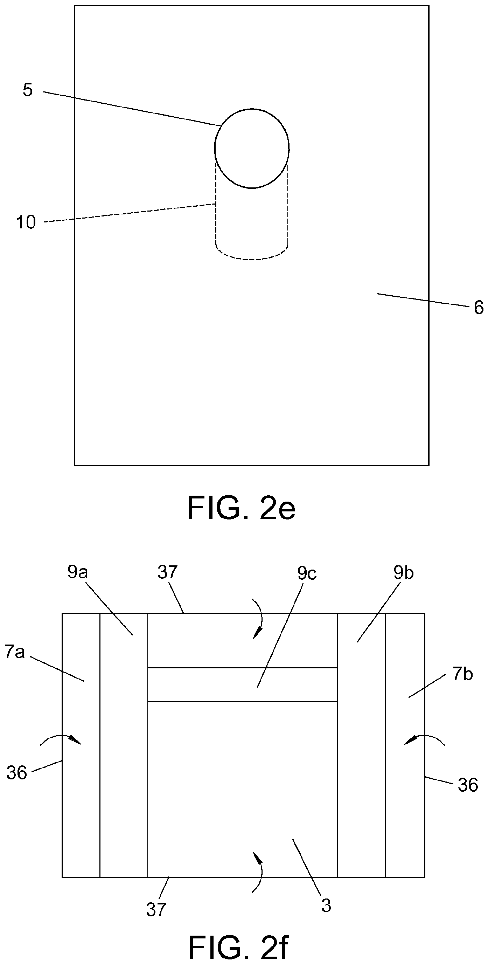

Figure 2.a' shows a side view of the "envelope type" absorbent diaper in figure 2.a, unfolded when manufactured, but which is configured for female use, wherein the hole (5') changes its position in the laminar body with respect to the hole (5) for anatomical reasons.
Figure 2.b shows a side view of the "envelope type" absorbent diaper with a cannula of figure 2.a, folded in a position of use forming a closed space on the inside thereof.
Figure 2.c shows a view of the front face (outer) of the "envelope type" absorbent diaper in figure 2.a, unfolded when manufactured.
Figure 2.d shows a view of the front face (outer) of the "envelope type" absorbent diaper in figure 2.a, unfolded when manufactured and with fastening bands with adhesive on the sides.
Figure 2.d' shows a view of the front face (outer) of the "envelope type" absorbent diaper in figure 2.d, but which is configured for female use, wherein the hole (5') changes its position in the laminar body with respect to the hole (5) for anatomical reasons.
Figure 2.e shows a view of the rear face (inner) of the "envelope type" absorbent diaper with a cannula in figure 2.a, folded in a position of use.
Figure 2.f shows a view of the front face (outer) of the "envelope type" absorbent diaper with a cannula in figure 2.a, folded in a position of use.

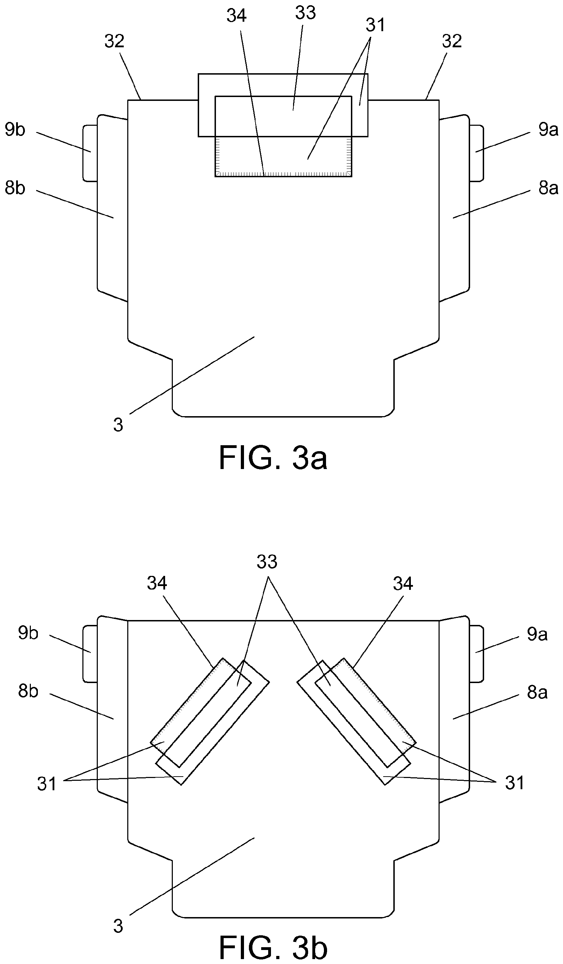

Figure 3.a shows a view of the front face (outer) of the "sealed type" absorbent diaper, with an opening and closing system in the front part that includes a protective absorbent core.
Figure 3.b shows a view of the front face (outer) of the "sealed type" absorbent diaper, with a double oblique frontal opening and closing system.

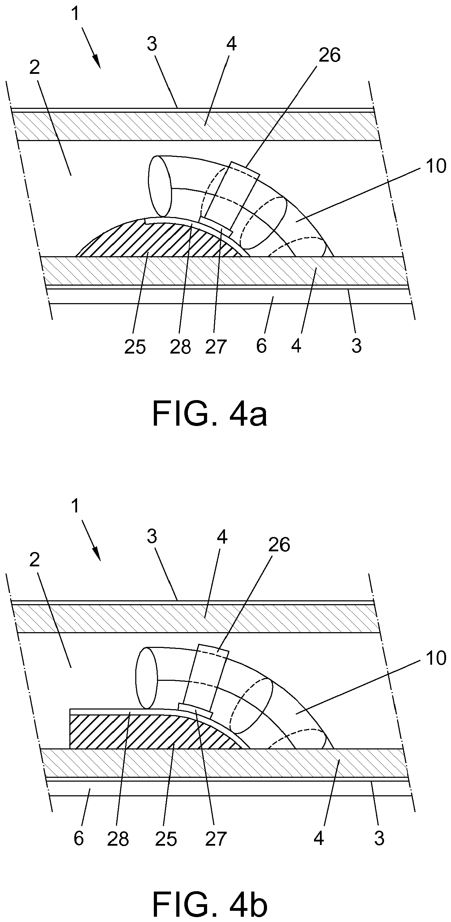

Figure 4.a shows a transverse cross section of the absorbent diaper: a detailed view of the inner container where the cannula is located, which rests on an absorbent core with a half-oval or semicylindrical form and is fastened to the same with an elongated and flat elastic strip with adhesive tape.
Figure 4.b shows a transverse cross section of the absorbent diaper: a detailed view of the inner container where the cannula is located, which rests on an absorbent core with a semicircular form at the end next to the lower part of the hole and is extended in a rectangular way at the other end, and is fastened to the same with an elongated and flat elastic strip with adhesive tape.

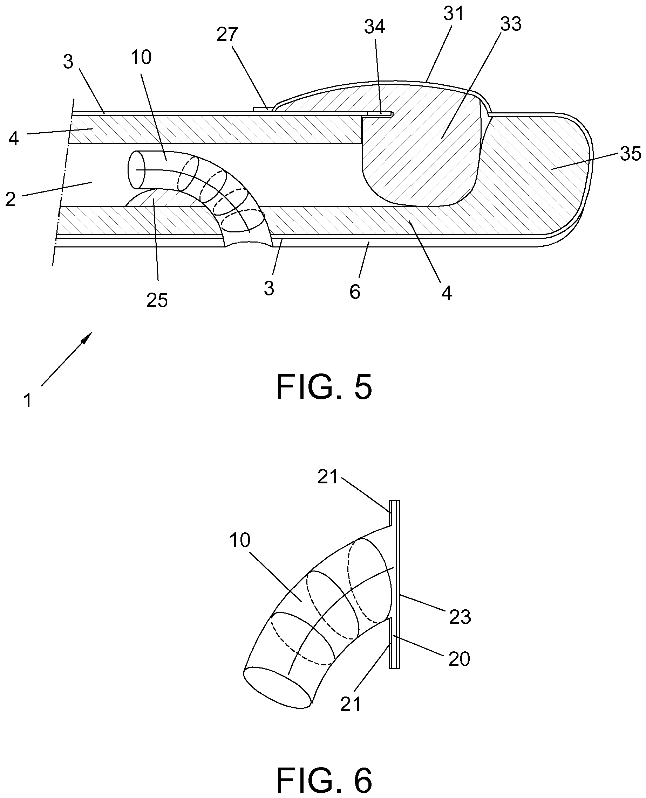

Figure 5 shows a transverse cross section of the absorbent diaper: the inner container where the cannula is located and the upper part with the opening and closing system with a protective double absorbent core. It also shows a detailed view of the opening and closing system which facilitates access to the inner container, with a double absorbent core and groove for coupling.
Figure 6 shows a side view of the cannula with an extension perpendicular to the longitudinal axis of the mouth thereof, with two faces: one with an adhesive and the other with a third layer that is smooth to the touch.

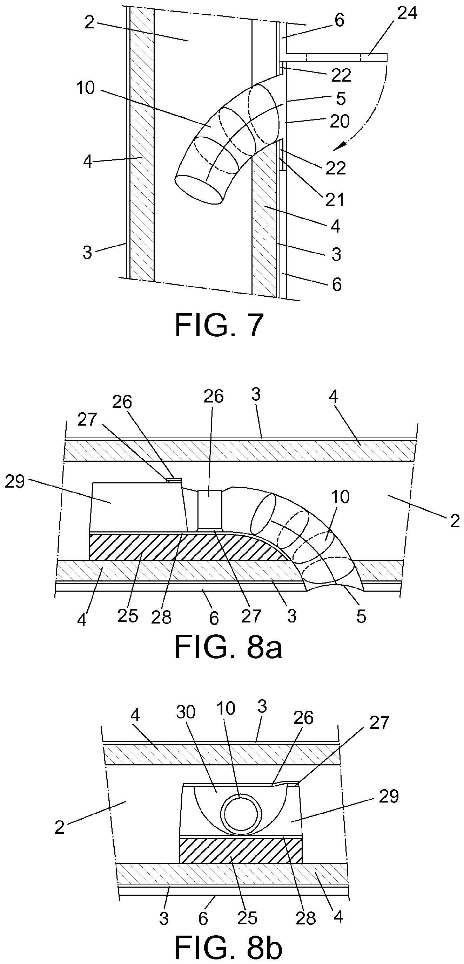

Figure 7 shows a transverse cross section of the absorbent diaper: a detailed view of the inner container where the cannula of figure 6 is located with an extension perpendicular to the longitudinal axis at the mouth thereof, introduced in the container through the hole and covered on the outer part thereof by a tab of the third layer of the diaper which functions like a window, the first impermeable layer further having an adhesive material around the hole to reinforce the connection with the flap of the cannula.
Figure 8.a shows a transverse cross section of the absorbent diaper: a detailed view of the inner container where the cannula is located, which has an absorbent core (with a semicircular form at the end next to the lower part of the hole and which is extended in a rectangular way at the other end) with a separating rigid structure that includes a U-shaped cavity where the cannula rests, and is fastened both to the absorbent core and to the separating structure by an elongated and flat elastic strip with adhesive tape.
Figure 8.b shows a bottom view of the transverse cross section of figure 8.a in which the cannula is seen inside the U-shaped cavity of the separating structure on the absorbent core.

### Preferred exemplary embodiments of the invention

The present invention particularly relates to two preferred embodiments that are detailed below.

The first of the embodiments consists of an absorbent diaper (see figure 1), made up of a flexible laminar body **(1)** folded over itself when manufactured joining the ends thereof and forming a sealed space on the inside thereof in form of a container **(2),** said body comprising at least:
- a first layer **(3),** made of an impermeable material which makes up the outer wall of the diaper and which is in contact with the skin and with the outside,
- a second layer **(4),** made of an absorbent material, adhered to the first layer on one of the two faces thereof and which partially or entirely covers the surface of said face, which makes up the inner wall of the container on part of, or all of, the perimeter thereof; and
- a hole **(5 or 5')** that passes through both layers **(3, 4)** and which, in a position of use, comes into contact with the urinary organ at the impermeable outer layer **(3),** such that it allows the fluids to enter the container **(2)** formed by the layers **(3, 4)** since the laminar body is folded over itself.

As can be seen in figure 1, a particular embodiment of this disposable absorbent diaper is intended for a male urinary organ such that said diaper is made up of a flexible laminar body **(1)** folded over itself when manufactured, joining the ends thereof and forming a sealed space on the inside thereof, in the form of a container **(2),** said body comprising at least:
- a first layer **(3),** made of an impermeable material which makes up the outer wall of the diaper and which is in contact with the skin and with the outside, and
- a second layer **(4),** made of an absorbent material, adhered to the first layer on one of the two faces thereof and which partially or entirely covers the surface of said face, which makes up the inner wall of the container on part of, or all of, the perimeter thereof; and
- a hole **(5)** which passes through both layers **(3, 4),** configured to allow for the introduction of the male urinary organ from the impermeable layer **(3)** to the absorbent layer **(4),** remaining inside the container **(2)** formed by the layers.

Another preferred embodiment of this disposable absorbent diaper is intended for a female urinary organ, specifically shown in figures 1.a' and 1.d', the diaper maintaining its configuration but, for anatomical reasons, the hole (5') is located in a different area of the laminar body **(1),** since it is adapted to this group of users (it is in or close to the central part of the laminar body).

The absorbent diaper described, in any of the variants thereof, can either be disposable (partially or completely) or reusable, depending on the configuration of the same.

This way, the body is folded in such a way that the impermeable layer **(3)** covers the outer part of the entire diaper on the two faces thereof, and on the inside of which it is adhered to the absorbent layer **(4)** which in turn delimits the perimeter of the container **(2).** In the cross-sectional view of the diaper in figure 1.a, the profile shows, from the outer face to the inner face of the diaper: (one part of) the impermeable layer adhered to (one part of) the absorbent layer, the inner space of the diaper, and (another facing part of the) absorbent layer adhered to (another facing part of the) impermeable layer. Since the flexible laminar body **(1)** is folded over itself, joined at the ends thereof, said folding forms a single diaper unit with two facing faces: one inner face, configured to come into contact with the user's body according to the position of use, and one outer face, which comes into contact with the environment and prevents fluid loss, each one of the faces being made up of the impermeable layer **(3)** as the outer wall and the absorbent layer **(4)** as an inner wall (see figure 1.b, wherein one can see the inside of the diaper, unfolded and divided into two walls, which when folded form the container or closed space **(2)).** The hole **(5 or 5'),** which therefore is on the inner face of the diaper (see figure 1.d and 1.d'), passes through the two layers **(3, 4)** and comes into contact with the urinary organ in a position of use. A shown in figure 1.a, the hole allows the male urinary organ to be introduced inside the container **(2),** where the urine is retained, while the inner face of the diaper serves as an area in which excrement is collected.

The diaper can have a third layer **(6)** that is smooth to the touch, from 1 to 5 millimeters thick, both limits included, adhered to the outer wall of the inner face of the diaper, which is the impermeable layer **(3)** intended to come into contact with the skin, which makes the use thereof more comfortable. This layer is made of a material selected from a group made up of cotton, polyester, bamboo fiber, hemp fiber or polypropylene, among the most important ones.

In addition to this third layer **(6),** the diaper can optionally include any of the previously described variants in the section titled *Detailed Description,* which are not essential for the functioning of the diaper but which do improve several aspects of the properties thereof, as was previously explained. Thus, the sides **(7a, 7b)** of the folded laminar body are heat-sealed in order to close the space between the inner and outer faces of the diaper, each one having one or two fastening bands **(8a, 8b, 8c, 8d),** like conventional diapers, which are elastic (adjustable) and which have an adhesive **(9a, 9b).** In turn, the second layer **(4)** of the laminar body **(1),** which is absorbent, has a relief surface with a series of longitudinal and transverse grooves **(13),** which make up a series of absorption centers **(14)** where the retention of the fluids is concentrated (see the details of figure 1.a).

In this first exemplary embodiment shown, and specifically in the case of being configured for the male urinary organ, the sealed type diaper incorporates a cannula **(10)** in the hole **(5)** in order to protect the skin inside the space **(2),** and an area around the hole **(5)** where part of the absorbent layer is covered with a layer (patch type) of an impermeable material **(11);** further including a second layer of an impermeable material **(12),** equivalent to the first, on the part of the absorbent layer **(4)** facing the area where the cannula is situated **(10).**

This cannula **(10),** as observed in detail in figure 1.b, has a layer made of an absorbent material **(15)** that coats the inside thereof, and which includes longitudinal bands or grooves **(16)** along the same, in the form of expansion joints. Likewise, the cannula **(10)** has several circular elastic strips **(17)** on the outer surface thereof, which adjust the diameter of said cannula **(10)** to the contours of the urinary organ without compressing it (see figure 1a). Alternatively, the cannula **(10)** has an elongated and flat elastic strip or band **(18),** joined to the surface at one point, such that it can roll up and the ends thereof can be joined by means of a Velcro-type opening and closing system **(19),** as shown in figure 1.b.

In an embodiment of the cannula **(10),** which is independent from the laminar body **(1)** and is shown in figure 6, the same has an extension **(20)** at its base or mouth that is perpendicular to the longitudinal axis of the cannula **(10),** with a flat form and with two faces, one of them, which can be called the front face due to the fact that it is configured to come into contact with the impermeable layer **(3),** having its surface covered by an adhesive element **(21)** in order to fasten said extension to said area around the hole **(5).** In this example, the area of the impermeable layer **(3)** around the hole **(5)** which in contact with the extension **(20)** of the mouth of the cannula **(10)** also includes an adhesive element **(22).** In one embodiment (see figure 6) the face of the extension **(20)** of the cannula **(10)** that comes into contact with the body (referred to as the rear body due to the fact that it is opposite that which has adhesive **(21)),** incorporates as a coating a soft absorbent strip **(23)** which is smooth to the touch, such as the one defined for the third layer **(6).** In another embodiment, as shown in figure 7, it is the third layer **(6)** which covers the outer face of the extension **(20)** of the mouth of the cannula **(10),** such that in this area said third layer **(6)** has a tab-like opening **(24)** or window-type opening thanks to a series of cuts, which closes after introducing the cannula **(10)** through the hole **(5),** covering the extension **(20)** of the mouth which remains outside of the hole **(5).**

The sealed type diaper illustrated in this example has an absorbent core **(25)** in the area of the absorbent layer **(4)** immediately below the hole **(5),** absorbent core that is an element made of an absorbent material, like that of the layer itself, or different, which adheres to the same in the form of a projection or protrusion on which the cannula **(10)** is supported in a position of use. In one variant (see figure 4.a), it has a semicylindrical surface (curved surface), while in another variant (see figure 4.b) said core **(25)** has a semicircular surface on the end near the lower part of the hole **(5)** and extends in the form of a rectangle at the other end. In one or another variant, the absorbent core **(25)** has a safety strap **(26)** in a crosswise direction with respect to the position of the organ or cannula **(10),** which is elastic and adhered at each one of the ends thereof to each of the sides of the core with a Velcro-type opening and closing system **(27).** Furthermore, the surface of the absorbent core **(25)** arranged to come into contact with the male urinary organ or with the cannula **(10)** is covered by an impermeable film **(28).** With the aim of preventing the compaction of the container **(2)** in the area where the cannula **(10)** is located once introduced in the hole, **(5)** the absorbent core **(25)** preferably incorporates a support **(29),** either rigid or flexible, which projects from the surface of the core **(25)** enough to be above the cannula **(10),** and in the central area thereof it has a U-shaped cavity **(30)** on which it rests against.

The example of the sealed type diaper includes an opening and closing system **(31)** of the inner container **(2)** (see figures 1.a and 1.b), which, among other functions, allows for the manipulation of the male urinary organ or the cannula **(10)** between the two inner and outer faces of the diaper. In one variant (see figure 3.a), said system **(31)** is placed on the upper end of the front part of the diaper where the laminar body **(1)** folds, and consists of a Velcro-type tab or a glued adhesive tab, situated in the central part of the width of the layers, in a horizontal position with respect to the length of the laminar body, which leaves, at the two sides thereof, part **(32)** of the laminar body **(1)** without the possibility of opening, with the aim that when the user is in a position in which they are lying down on their side, the fluid retained inside does not spill over the ends. In another variant (see figure 3.b), the opening and closing system **(31)** consists of one or two Velcro-type tabs located on the front outer face of the laminar body **(1)** according to the position of use, facing the part of the inner face where the hole **(5)** is located. In a third variant, the opening and closing system consists of an opening with a tab in a vertical position, in other words, perpendicular with respect to the end of the diaper that forms the waistline, situated in the front part of the diaper in a position of use, facing the part of the inner face where the hole is located, or moved towards either of the sides with respect to the hole and a the same height. In any of these cases, the inside of the tab, which when closed remains inside the container **(2),** is covered on the entire surface thereof by a strip with a thickness from 1 to 5 millimeters, both limits included, of an absorbent material similar to the absorbent layer **(4),** and is covered by, or consists of, a semicylindrical absorbent core **(33),** similar to the one that was previously described, which has a greater thickness than that of the absorbent layer **(4)** of the laminar body **(1)** and which allows the two facing faces of the absorbent layer to be joined by contact so as to prevent the passage of fluid through that area. Moreover, to adhere the opening and closing system **(31)** to the absorbent core **(33)** and prevent gaps from existing between both parts, the opening of the tab system can incorporate an elastic edge **(34).** Also, with the same objective, the absorbent core **(33)** that covers the inner surface of the tab includes a groove on the edge thereof, where part of the impermeable layer **(3)** is introduced when closed, with the aim of said groove or edge better sealing the closure to elastic band of the opening and preventing leaks.

When the tab of the opening and closing system **(31)** is located on the upper part of the front face of the diaper, the absorbent layer **(4)** of the body **(1)** that covers the inside of the container **(2)** has an additional absorbent core **(35)** arranged crosswise to the length of the layers **(3, 4)** and equal to the width of the same, in a semicylindrical or half-oval form, at the same height or above said opening and closing system **(31).** This absorbent core **(35)** consists of an increase in the thickness of the absorbent layer **(4)** in the upper area of the diaper according to the position of use (around the waistline), enough to join the two faces of the absorbent layer **(4)** of the folded body **(1)** by contact in the form of a projection or protrusion that forms an absorbent strap with the core **(33)** of the tab of the opening and closing system **(31),** since both are in contact when the tab closes, sealing the upper area from the passage of liquid and humidity.

This way, the two sides **(32)** of the opening and closing system **(31)** are completely covered by an absorbent material.

The second of the preferred embodiments of the invention consists of an absorbent dressing/diaper (see figure 2) that is an "envelope type", made up of an extended flexible laminar body **(1)** (unfolded when manufactured), which comprises:
- a first layer **(3),** made of an impermeable material, and
- a second layer **(4),** made of an absorbent material, adhered to the first layer on one of the two faces thereof and which partially or entirely covers the surface of said face,
configured to fold over itself when placed on the user's body, delimiting a closed inner space, in the form of a container **(2),** such that the impermeable layer **(3)** is an outer wall in contact with the skin and with the environment and the absorbent layer **(4)** is the inner wall of the space in all of, or part of, the inner perimeter of the space; and
- a hole **(5 or 5')** that passes through both layers **(3, 4)** and which, in a position of use, comes into contact with the urinary organ at the impermeable layer **(3),** such that it allows the fluids to enter the container **(2)** formed by the layers **(3, 4)** when the laminar body is folded over itself.

As can be seen in figure 2, a particular embodiment of this "envelope type" disposable absorbent diaper is intended for a male urinary organ, such that said diaper is made up of an extended flexible laminar body **(1)** which comprises:
- a first layer **(3),** made of an impermeable material, and
- a second layer **(4),** made of an absorbent material, adhered to the first layer on one of the two faces thereof and which partially or entirely covers the surface of said face,
configured to fold over itself when placed on the user's body, delimiting a closed inner space, in the form of a container **(2),** such that the impermeable layer **(3)** is an outer wall in contact with the skin and with the environment and the absorbent layer **(4)** is the inner wall of the space in all of, or part of, the inner perimeter of the space; and
- a hole **(5)** which passes through both layers **(3, 4),** configured to allow for the introduction of the male urinary organ from the impermeable layer **(3)** to the permeable layer **(4),** remaining within the closed space **(2)** when the flexible body is folded.

In another preferred embodiment of this disposable absorbent diaper intended for a female urinary organ, specifically shown in figures 2.a' and 2.d', the diaper maintains its configuration but, for anatomical reasons, the hole (5') is located in a different area of the laminar body **(1)** since it is adapted to this group of users (it is in, or close to, the central part of the laminar body).

This absorbent dressing/diaper described can either be disposable (partially or completely) or reusable, depending on the configuration of the same.

Unlike the previous embodiment, the laminar body **(1)** that makes up the diaper is extended when manufactured, and once placed on the user's body, in such a way that it comes into contact with the urinary organ at the impermeable outer layer (preferably, the male urinary organ is introduced in the hole **(5)),** and ready for use, it is folded over itself towards the front part of the body, the ends thereof being joined, in the form of an envelope. This way, as can be seen in figure 2.b, when the flexible laminar body **(1)** is folded, the ends thereof being joined, it is sealed by means of two adhesive bands, or by means of separable joining means (which can be, for example, made of a Velcro-type material) on the sides **(9a, 9b)** on the entire length thereof, and an upper band **(9c),** which can be, just as on the sides **(9a, 9b),** an adhesive or with separable joining means (for example, a Velcro opening and closing system) on the two ends thereof, which joins the ends of the body **(1)** when folded, said fold forming a diaper with two facing faces: one inner face, configured to come into contact with the user's body according to the position of use, and one outer face in contact with the outside, each one of the faces made up of the impermeable layer **(3)** as an outer wall and the absorbent layer **(4)** as an inner wall.

In this way, the diaper covers the front part of the body, and is intended to collect the urine. It is possible, in a preferable case, when used for male urinary organs and, above all, when used for female organs, that the laminar body **(1)** of the disposable absorbent diaper has an extended configuration, preferably an elongated rectangular configuration, with a length such that, once folded and closed towards the front to form the container **(2),** the lower part thereof can fold between the user's legs towards their back, like a normal diaper, going around their body; once in this position, it can be fastened to the body with a fastening system that joins the upper part of the diaper to the lower part around the waist of the user, according to the position of use (see figures 2.d and 2.d'), such as a system of straps, adhesive or similar, such as the bands of conventional diapers or similar to the fastening bands **(8a, 8b, 8c, 8d)** of the sealed type diaper that are elastic (adjustable) and have adhesive **(9a, 9b).** It is also possible to combine (or fasten) this envelope type diaper with a common and standard diaper known in the state of the art, which at the same time retains the feces; this combination making it possible to discard the common diaper with the waste from defecation and keep the diaper object of the present invention on the user's body, since it usually has a longer use when used only for liquid fluids, and not for both.

As was previously stated, the upper band **(9c)** (adhesive or, alternatively, a Velcro-type system or another type of separable system) is located at the upper end of the laminar body **(1)** according to the position of use, such that it is joined to the other end making up the inner container **(2)** by folding, also sealing the sides thereof **(7a, 7b)** with the longitudinal adhesive bands **(9a, 9b)** (or with another type of fixed or separable joint, such as a Velcro-type joint). To be able to laterally fold and close in an easy way, in addition to the adhesive bands **(9a, 9b, 9c),** the flexible laminar body **(1)** has longitudinal folding areas **(36)** to seal the sides around the body, as can be seen in figure 2.c, and crosswise folding areas **(37),** on which the layers **(3, 4)** are folded, joining the ends thereof to form the container **(2).**

In a preferred case, the face of the impermeable layer **(3)** opposite that which is adhered to the absorbent layer **(4)** has, in turn, a third layer **(6)** of a material smooth to the touch, adhered to the same and which can either have absorbent properties or be a washable fabric that covers part of, or all of, the surface thereof, with a thickness from 1 to 5 millimeters, both limits included, and which will be both on the outer face as well as on the inner face of the diaper (in contact with the user) according to the embodiment, unless a different composition is given to each part (the outer part and the inner part in contact with the user's body). In addition to this third layer **(6),** the diaper can optionally include any of the previously described variants in the section titled *Detailed Description,* which are not essential for the functioning of the diaper but which do improve several aspects of the properties thereof. Thus, the second layer (4) of the laminar body, which is absorbent, has a relief surface with a series of longitudinal and transverse grooves **(13),** which make up a series of absorption centers **(14)** where the retention of the fluids is concentrated.

The details of the invention that are related to the cannula that were previously described for the first preferred embodiment of the invention, the sealed type diaper, when the diaper is configured for the male urinary organ, are also applicable to the envelope type diaper. Thus, the sealed type diaper in this example incorporates a cannula **(10)** in the hole **(5)** in order to protect the skin inside the space, and an area around the hole **(5)** where part of the absorbent layer **(4)** is covered with a layer (patch type) of an impermeable material **(11);** further including a second layer of an impermeable material **(12),** equivalent to the first, on the part of the absorbent layer **(4)** which is facing the cannula **(10)** when the diaper is folded, forming the container **(2).**

This cannula **(10)** has all of the previously described characteristics in the case of the sealed type diaper: a layer of an absorbent material **(15)** that covers the inside thereof, and which includes longitudinal bands or grooves **(16)** along the same, in the form of expansion joints; circular elastic strips **(17)** on the outer surface thereof which adjust the diameter of the cannula to the contours of the urinary organ without compressing it (see figure 2a); alternatively, it can include an elongated and flat elastic strip **(18)** which can be joined at one point to the surface of the cannula **(10)** or completely separated, such that it can roll around the same and the ends thereof can be joined by means of a Velcro-type opening and closing system **(19),** among others, as well as an extension **(20)** at the base or mouth thereof, perpendicular to the longitudinal axis of the cannula **(10)** and in a flat form with two faces, one of which can be referred to as front due to the fact that it is configured to come into contact with the impermeable layer **(3),** the surface of which is covered by an adhesive element **(21)** in order to fasten said extension to the area around the hole **(5).** Also, the area of the impermeable layer **(3)** around the hole **(5)** which in contact with the extension **(20)** of the mouth of the cannula **(10)** includes an adhesive element **(22).** The face of the extension **(20)** of the cannula **(10)** that comes into contact with the body (referred to as rear, since it is opposite to that which has adhesive **(21)),** incorporates an absorbent strip **(23)** as a cover that is smooth to the touch, such as that which is defined for the third layer **(6),** or alternatively, it is the third layer **(6)** which covers the outer face of the extension **(20)** of the cannula **(10),** such that in this area said third layer **(6)** has a tab-like opening **(24)** or window-type opening thanks to a series of cuts, which closes after introducing the cannula **(10)** through the hole **(5),** covering the extension **(20)** of the mouth which remains outside of the hole **(5).**

Also like the sealed type diaper, the envelope type dressing/diaper illustrated in this example preferably has an absorbent core **(25)** in the area of the absorbent layer **(4)** immediately below the hole **(5),** which adheres to the same in the form of a projection or protrusion on which the cannula **(10)** is supported in a position of use. As in the previous example, it can take any of the described forms and any of the defined fastening means of the cannula **(10),** such as the safety strap **(26)** in a crosswise direction with respect to the position of the organ or cannula **(10),** which is elastic and adhered at each one of the ends thereof to each of the sides of the core with a Velcro-type opening and closing system **(27).** Furthermore, the surface of the absorbent core **(25),** arranged to come into contact with the cannula **(10),** is covered by an impermeable film **(28).** With the aim of preventing the compaction of the container **(2)** in the area where the cannula **(10)** is located, once introduced in the hole **(5),** the absorbent core **(25)** incorporates a support **(29),** either rigid or flexible, which projects from the surface of the core **(25)** enough to be above the cannula **(10),** and in the central area thereof has a U-shaped cavity **(30)** on which the same rests against.

In both preferred embodiments that are described in this section, called sealed-type diaper and envelope-type dressing/diaper, it is possible that the second layer, which is absorbent and retains fluids, is adhered to the first, impermeable layer on one of the faces thereof by means of separable joining means which are Velcro-type, separable and adhesive, or similar. This allows both layers to be separated, such that the absorbent layer can be removed after the use thereof in order to be washed and used again in the diaper, being adhered again to the impermeable layer. It is also possible to extract and discard said used absorbent layer and replace it with a new layer that has similar characteristics. Since the layers are adhered to each other by means of separable joining means, the simplicity of said configuration makes it so that it can be the user who manipulates both layers before and after use in an easy way. Likewise, in the case of the sealed diaper, the absorbent layer that is inside the diaper can be extracted or adhered through the opening or openings included. On the other hand, in the envelope-type dressing/diaper it is possible to unfold the laminar body to remove or adhere the absorbent layer when it is extended.

In this particular case, described for the two main examples, where the first and second layers are adhered by separable joining means, they can be distributed on facing faces of both layers that come into contact in multiple ways, such that they guarantee a proper fastening.

Specifically, the absorbent anatomical article that also retains fluids and waste from the human body described in the present specification, in any of its variants and particularly these two examples herein described, can be: disposable, partially disposable (only when the absorbent layer is extracted and not reused) or reusable (if the absorbent layer is removed and washed and the impermeable layer is then adhered again).

The fact that these examples of the article are reusable is favored when the third layer, smooth to the touch, covers the entire outer part of the impermeable layer to which it is adhered, and is made up of a washable material (cotton, polyester, hemp or bamboo fibers, etc.) which, in the first place, allows the absorbent layer to be separated from the impermeable layer in order to be washed, and, furthermore, allows the outer layer of the laminar body to be washed, the laminar body being formed by the third layer that is smooth to the touch. Moreover, this possible separation of layers allows the impermeable layer and absorbent layer to be independently provided and distributed, being able to create several different models of each layer separately, and providing the user with the possibility of acquiring the ones that meet their needs, in addition to providing them with the possibility of discarding and recycling the layers separately.

## Claims

1. An anatomical absorbent article having retaining properties for fluids and waste of the human body, which comprises a single flexible laminar body (1) that comprises:
- a first layer (3), made of an impermeable material,
- a second layer (4), made of an absorbent material, adhered to the first layer (3) on one of the two faces thereof and which partially or entirely covers the surface of said face,
said laminar body (1) being configured to fold over itself and delimit a closed inner space, in the form of a container (2), such that the impermeable layer (3) constitutes the outer wall in contact with the user's skin and the absorbent layer (4) constitutes the inner wall of the container (2) in all of, or part of, the inner perimeter thereof; and
- a hole (5, 5') that passes through both layers (3, 4) and which, in a position of use, comes into contact with the urinary organ at the impermeable outer layer (3), such that it allows the fluids to enter the container (2) when the laminar body (1) is folded over itself.

2. The anatomical absorbent article having retaining properties for fluids and waste of the human body according to claim 1, wherein the hole (5, 5') that passes through both layers (3, 4) and that comes into contact with the urinary organ in a position of use is intended to allow for the urinary organ to be introduced from the impermeable outer layer (3) to the absorbent inner layer (4), such that the urinary organ remains inside the container (2) when the body (1) is folded over itself.

3. The anatomical article of any one of claims 1 or 2, which is disposable.

4. The anatomical article of any one of the preceding claims, wherein part of the face of the impermeable layer (3) opposite the face where the absorbent layer (4) is adhered has a third, covering layer (6) adhered that is smooth to the touch, able to be in contact with the user's body, or wherein the entire face of the impermeable layer (3) opposite the face where the absorbent layer (4) is adhered has said third layer (6) adhered, which covers all of the outer surface of the anatomical article, both on the outer face thereof in contact with the environment and on the inner face thereof which is in contact with the user's body in a position of use.

5. The anatomical article of any of the preceding claims, wherein the second, absorbent layer (4) has a relief surface with a system of channels (13) in a grid-like form through which the fluid is distributed inside the container (2) and which delimits cores (14) of the absorbent layer (4) where the absorption of said fluids is concentrated.

6. The anatomical article of any of the preceding claims, wherein the inner face of the second, absorbent layer (4), is covered by a layer made of an impermeable material (11, 12) that is the same as or different from that of the first impermeable layer (3) of the laminar body (1) in two areas: the area around the hole (5, 5') and the area that is facing the hole (5, 5') when the laminar body (1) is folded over itself.

7. The anatomical article of any of the preceding claims 2 to 6, wherein the laminar body (1) incorporates a cannula (10) made of an elastic and flexible impermeable material that passes through the layers (3, 4) by the hole (5, 5'), intended to cover the male urinary organ of the user.

8. The anatomical article of the preceding claim, wherein the cannula (10) incorporates a flat elastic band (18) on the outer surface thereof with a length sufficient to surround said cannula (10), with an opening and closing system (19) at the two ends thereof.

9. The anatomical article of any of the claims 7 or 8, wherein the cannula (10) has a layer made of an absorbent material (15) on the inner face thereof, able to be in contact with the user, in the form of longitudinal bands along the axis of the cannula (10) separated by grooves (16) that make an expansion joint in order to allow for the expansion of the cannula (10) when introducing the urinary organ, without limiting the elasticity or flexibility thereof and without choking the urinary organ.

10. The anatomical article of any of the claims 7 to 9, wherein the cannula (10) is independent from the laminar body (1) and has a flat extension (20) with two faces in the mouth thereof, perpendicular with respect to the longitudinal axis of said cannula (10), wider than the diameter thereof in order to form a stop, preventing the cannula (10) from being completely introduced inside the container (2); and wherein:
- the front face of the extension (20) that is in contact with the impermeable layer (3) of the article has an adhesive or separable joining means (21) between the two to fasten said extension to the area around the hole (5, 5') that is covered by the extension (20); and
- the rear face of the extension (20) able to come into contact with the user's body incorporates on the surface thereof an absorbent covering layer (23) that is smooth to the touch for thereby increasing comfort.

11. The anatomical article of any of the preceding claims 2 to 10, wherein the face of the second, absorbent layer (4), which makes up the inner wall of the container (2) when the laminar body (1) is folded over itself incorporates a core (25) in the area that is immediately below the hole (5, 5'), according to the position of use, in which the urinary organ is supported, said core (25) being formed by an increase in the thickness of the absorbent layer (4) in the area situated below the hole (5, 5') or by an independent element, of an absorbent material or of an insulating material, which adheres to the absorbent layer (4) in that area, in the form of a projection or protrusion; said core (25) comprising an elastic safety strap (26) in an crosswise direction with respect to the longitudinal position of the organ on the core (25), which has an opening and closing system (27) on each one of the two sides thereof to remain joined to both sides of the core (25) and to fasten the organ.

12. The anatomical article of the preceding claim, wherein when the core (25) is made of an absorbent material, said core (25) includes an impermeable coating film (28) on the surface thereof that comes into contact with the urinary organ in order to prevent the accumulation of humidity in said area and to allow for the absorption on the sides of the core (25) that are not coated by the impermeable film (28).

13. The anatomical article of any of the claims 11 or 12, wherein the core (25) has on the surface thereof a rigid or flexible separating structure (29) that projects above the urinary organ able to rest on said core (25), intended to ensure a hollowing in the area of the container (2) around the hole (5, 5') and prevent compaction, and which has a hollow inner central area (30), in which said organ is housed, providing a barrier and support to the same.

14. The anatomical article of any of the preceding claims, which is an absorbent diaper wherein the flexible laminar body (1) is unfolded when manufactured and has crosswise and longitudinal folding areas, being configured to fold over itself towards the front in the form of an envelope in a position of use when placed on the user's body, joining the ends of the laminar body (1) by means of an opening and closing system (9c) that enables it to be sealed and thereby delimiting the inner container (2), and wherein the sides (7a, 7b) of the laminar body (1) have adhesive bands or separable joining means (9a, 9b) along the entire length thereof in order to seal the container (2) once folded.

15. The anatomical article of the preceding claim, wherein the laminar body (1) has an elongated configuration with a length such that, once it is folded and closed towards the front to form the container (2), the lower part thereof according to the position of use is folded between the legs of the user towards their back, going around their body.

16. The anatomical article of any of the claims 1 to 13, which is an absorbent diaper wherein the flexible laminar body (1) is folded over itself when manufactured, being joined at the ends thereof and the sealed sides forming the container (2).

17. The anatomical article according to any of the claims 14 to 16, which has at least one opening and closing system (31) of the container (2), said opening and closing system (31) being located in an area of the laminar body (1) located above the hole (5, 5') that passes through the layers (3, 4).

18. The anatomical article of the preceding claim, wherein the opening and closing system (31) is located in the central region of the upper end of the diaper, at the height of the waistline of the user according to the position of use, said system being flanked on both sides by the laminar body (1); or at any other point of the part of the laminar body (1) that is facing the hole (5, 5') when folded in a position of use, between the height of the hole (5, 5') and of said upper end of the diaper.

19. The anatomical article of any of the claims 14 to 16, which has an opening and closing system (31) for the container (2) situated in the area of the laminar body (1) that is facing the part where the hole (5, 5') is housed when it is folded in a position of use, in a vertical position perpendicular with respect to the end of the diaper that forms the waistline in a position of use and at the same height of the hole (5, 5'), in front of the same or moved to a side position.

20. The anatomical article of the any of the claims 14 to 16, which comprises two opening and closing systems (31) located on the front face of the diaper, above the position of the hole (5, 5') and below the upper end of the laminar body (1) in a position of use, said systems (31) being in an oblique position with respect to the length of the folded laminar body (1) to facilitate manipulation.

21. The anatomical article of any of the claims 17 to 20, wherein the opening and closing system (31) is an elongated opening in the laminar body (1) with an adhesive tab, a Velcro-type tab or separable joining means, such that once it is closed, it covers said opening, sealing it, and wherein said tab incorporates an absorbent layer that covers the entire inner face thereof and remains inside the container (2), preventing the passage of liquid into the inner area of the tab, and said absorbent layer incorporates an absorbent core (33) on the surface thereof which has a thickness greater than that of the second, absorbent layer (4) of the laminar body (1) and sufficient to join by contact the two facing faces of the absorbent layer (4) of the folded laminar body (1).

22. The anatomical article of any one of the preceding claims, wherein the second layer (4) is adhered to the first layer (3) on one of the two faces thereof by separable joining means.

## Patentansprüche

1. Anatomischer absorbierender Artikel mit Rückhalteeigenschaften für Flüssigkeiten und Ausscheidungen des menschlichen Körpers umfassend einen einzelnen flexiblen geschichteten Körper (1, der umfasst:
eine erste Schicht (3), die aus einem undurchlässigen Material besteht, eine zweite Schicht (4), die aus einem absorbierenden Material besteht, an der ersten Schicht (3) an einer der zwei Seiten derselben haftet und die Oberfläche der Seite teilweise oder
vollständig abdeckt, wobei der geschichtete Körper (1) so ausgeführt ist, dass er umgeschlagen wird und einen geschlossenen Innenraum in Form eines Behälters (2) so begrenzt, dass die undurchlässige Schicht (3) die Außenwand bildet, die in Kontakt mit der Haut des Benutzers ist, und die absorbierende Schicht (4) die Innenwand des Behälters (2) über den gesamten Innenumfang oder einen Teil desselben bildet; sowie
ein Loch (5, 5'), das durch beide Schichten (3, 4) hindurch verläuft und das in einer Einsatzposition an der undurchlässigen äußeren Schicht (3) so in Kontakt mit dem Harnorgan kommt, dass es zulässt, dass die Flüssigkeiten in den Behälter (2) gelangen, wenn der geschichtete Körper (1) umgeschlagen ist.

2. Anatomischer absorbierender Artikel mit Rückhalteeigenschaften für Flüssigkeiten und Ausscheidungen des menschlichen Körpers nach Anspruch 1, wobei das Loch (5, 5'), das durch beide Schichten (3, 4) hindurch verläuft und in einer Einsatzposition mit dem Harnorgan in Kontakt kommt, dazu dient, zuzulassen, dass das Harnorgan über die undurchlässige äußere Schicht (3) zu der absorbierenden inneren Schicht (4) so eingeführt wird, dass das Harnorgan im Inneren des Behälters (2) verbleibt, wenn der Körper (1) umgeschlagen wird.

3. Anatomischer Artikel nach einem der Ansprüche 1 oder 2, der ein Einwegartikel ist.

4. Anatomischer Artikel nach einem der vorangehenden Ansprüche, wobei an einem Teil der Seite der undurchlässigen Schicht (3), die der Seite gegenüberliegt, an der die absorbierende Schicht (4) haftet, eine dritte, abdeckende Schicht (6) haftet, die sich glatt anfühlt und mit dem Körper des Benutzers in Kontakt kommen kann, oder wobei an der gesamten Seite der undurchlässigen Schicht (3), die der Seite gegenüberliegt, an der die absorbierende Schicht (4) haftet, die dritte Schicht (6) haftet,
die die gesamte Außenfläche des anatomischen Artikels sowohl an der Außenseite, die mit der Umgebung in Kontakt kommt, als auch an der Innenseite abdeckt, die in einer Einsatzposition mit dem Körper des Benutzers in Kontakt ist.

5. Anatomischer Artikel nach einem der vorangehenden Ansprüche, wobei die zweite, absorbierende Schicht (4) eine Relief-Fläche mit einem System von Kanälen (13) in einer gitterartigen Form aufweist, über die die Flüssigkeit im Inneren des Behälters (2) verteilt wird und die Kerne (14) der absorbierenden Schicht (4) abgrenzt, in denen sich die Absorption der Flüssigkeiten konzentriert.

6. Anatomischer Artikel nach einem der vorangehenden Ansprüche, wobei die Innenseite der zweiten, absorbierenden Schicht (4) durch eine Schicht, die aus einem undurchlässigen Material (11, 12) besteht, das das gleiche ist wie oder ein anderes als das der ersten undurchlässigen Schicht (3) des geschichteten Körpers (1) in zwei Bereichen abgedeckt wird, d.h. dem Bereich um das Loch (5, 5') herum und dem Bereich, der dem Loch (5, 5') zugewandt ist, wenn der geschichtete Körper (1) umgeschlagen ist.

7. Anatomischer Artikel nach einem der vorangehenden Ansprüche 2 bis 6, wobei der geschichtete Körper (1) eine aus einem elastischen und flexiblen undurchlässigen Material bestehende Röhre (10) einschließt, die über das Loch (5, 5') durch die Schichten (3, 4) hindurch verläuft und dazu dient, das männliche Harnorgan des Benutzers abzudecken.

8. Anatomischer Artikel nach dem vorangehenden Anspruch, wobei die Röhre (10) an ihrer Außenfläche ein flaches elastisches Band (18) mit einem System (19) zum Öffnen und Verschließen an ihren zwei Enden einschließt, dessen Länge ausreicht, um die Röhre (10) zu umschließen.

9. Anatomischer Artikel nach einem der Ansprüche 7 oder 8, wobei die Röhre (10) an ihrer Innenseite eine aus einem absorbierenden Material (15) bestehende Schicht, die mit dem Benutzer in Kontakt kommen kann, in Form von Längsbändern entlang der Achse der Röhre (10) aufweist, die durch Rillen (16) getrennt sind, die einen Dehnungsausgleicher bilden, um die Ausdehnung der Röhre (10) beim Einführen des Harnorgans zuzulassen, ohne die Elastizität oder Flexibilität derselben einzuschränken und ohne das Harnorgan zu quetschen.

10. Anatomischer Artikel nach einem der Ansprüche 7 bis 9, wobei die Röhre (10) unabhängig von dem geschichteten Körper (1) ist und eine flache Verlängerung (20) mit zwei Flächen in ihrer Öffnung aufweist, die senkrecht in Bezug auf die Längsachse der Röhre (10) und breiter als ihr Durchmesser ist und einen Anschlag bildet, der verhindert, dass die Röhre (10) vollständig in das Innere des Behälters (2) eingeführt wird; und wobei:
- die Vorderseite der Verlängerung (20), die in Kontakt mit der undurchlässigen Schicht (3) des Artikels ist, eine haftende oder lösbare Verbindungseinrichtung (21) zwischen den beiden aufweist, mit der die Verlängerung an dem Bereich um das Loch (5, 5') herum befestigt wird, der von der Verlängerung (20) abgedeckt wird; und
- die Rückseite der Verlängerung (20), die mit dem Körper des Benutzers in Kontakt kommen kann, an ihrer Oberfläche eine absorbierende abdeckende Schicht (23) einschließt, die sich glatt anfühlt und so den Komfort erhöht.

11. Anatomischer Artikel nach einem der vorangehenden Ansprüche 2 bis 10, wobei die Seite der zweiten, absorbierenden Schicht (4), die die Innenwand des Behälters (2) bildet, wenn der geschichtete Körper (1) umgeschlagen ist, einen Kern (25) in dem Bereich einschließt, der sich entsprechend der Einsatzposition, in der das Harnorgan aufgenommen ist, unmittelbar unter dem Loch (5, 5') befindet, der Kern (25) durch eine Zunahme der Dicke der absorbierenden Schicht (4) in dem unter dem Loch (5, 5') befindlichen Bereich oder durch ein unabhängiges Element aus einem absorbierenden Material oder einem isolierenden Material, das an der absorbierenden Schicht (4) in diesem Bereich haftet, in Form eines Vorsprungs oder einer Ausbuchtung gebildet wird, wobei der Kern (25) einen elastischen Sicherheitsriemen (26) in einer Querrichtung in Bezug auf die Längsposition des Organs an dem Kern (25) umfasst, der ein System (27) zum Öffnen und Verschließen an jeder der zwei Seiten desselben aufweist, damit er mit beiden Seiten des Kerns (25) verbunden bleibt und das Organ befestigt.

12. Anatomischer Artikel nach dem vorangehenden Anspruch, wobei, wenn der Kern (25) aus einem absorbierenden Material besteht, der Kern (25) einen undurchlässigen Beschichtungsfilm (28) an seiner Fläche aufweist, die mit dem Harnorgan in Kontakt kommt, um die Ansammlung von Feuchtigkeit in dem Bereich zu verhindern und die Absorption an den Seiten des Kerns (25) zuzulassen, die nicht mit dem undurchlässigen Film (28) beschichtet sind.

13. Anatomischer Artikel nach einem der Ansprüche 11 oder 12, wobei der Kern (25) an seiner Oberfläche eine starre oder flexible Trennstruktur (29) aufweist, die über das Harnorgan vorsteht, das auf dem Kern (25) aufliegen kann, und dazu dient, eine Aushöhlung in dem Bereich des Behälters (2) um das Loch (5, 5') herum zu gewährleisten und Verdichtung zu verhindern, und die einen hohlen inneren Mittelbereich (30) aufweist, in dem das Organ aufgenommen wird und der eine Barriere und Stütze dafür bildet.

14. Anatomischer Artikel nach einem der vorangehenden Ansprüche, der eine absorbierende Windel ist, wobei der flexible geschichtete Körper (1) beim Herstellen aufgeklappt ist und Quer- und Längs-Faltbereiche aufweist, so ausgeführt ist dass er in einer Einsatzposition in Form eines Umschlags zu der Vorderseite hin umgeschlagen wird, wenn er auf den Körper des Benutzers aufgelegt wird, die Enden des geschichteten Körpers (1) mittels eines Systems (9c) zum Öffnen und Verschließen verbunden werden, das es ermöglicht, ihn abzudichten und damit den inneren Behälter (2) abzugrenzen, und wobei die Seiten (7a, 7b) des geschichteten Körpers (1) Klebestreifen oder lösbare Verbindungseinrichtungen (9a, 9b) über ihre gesamte Länge aufweisen, um den Behälter (2) nach dem Falten abzudichten.

15. Anatomischer Artikel nach dem vorangehenden Anspruch, wobei der geschichtete Körper (1) eine längliche Form mit einer Länge hat, durch die, wenn er gefaltet und zu der Vorderseite hin geschlossen ist und den Behälter (2) bildet, der untere Teil desselben entsprechend der Einsatzposition zwischen den Beinen des Benutzers zu seinem Rücken hin gefaltet ist, so dass er um seinen Körper herum reicht.

16. Anatomischer Artikel nach einem der Ansprüche 1 bis 13, der eine absorbierende Windel ist, wobei der flexible geschichtete Körper (1) beim Herstellen umgeschlagen wird, er an seinen Enden sowie den abgedichteten Seiten verbunden wird und den Behälter (2) bildet.

17. Anatomischer Artikel nach einem der Ansprüche 14 bis 16, der wenigstens ein System (31) zum Öffnen und Verschließen des Behälters (2) aufweist, wobei sich das System (31) zum Öffnen und Verschließen in einem Bereich des geschichteten Körpers (1) befindet, der oberhalb des Lochs (5, 5') liegt, das durch die Schichten (3, 4) hindurch verläuft.

18. Anatomischer Artikel nach dem vorangehenden Anspruch, wobei sich das System (31) zum Öffnen und Verschließen entsprechend der Einsatzposition in dem Mittelbereich des oberen Endes der Windel in Höhe der Taille des Benutzers befindet, und das System auf beiden Seiten oder an jedem anderen Punkt des Teils des geschichteten Körpers (1), der dem Loch (5, 5') zugewandt ist, wenn er in einer Einsatzposition gefaltet ist, zwischen der Höhe des Lochs (5, 5') und des oberen Endes der Windel durch den geschichteten Körper (1) seitlich begrenzt wird.

19. Anatomischer Artikel nach einem der Ansprüche 14 bis 16, der ein System (31) zum Öffnen und Verschließen für den Behälter (2), das sich in dem Bereich des geschichteten Körpers (1) befindet, der dem Teil zugewandt ist, in dem das Loch (5, 5') aufgenommen ist, wenn er in einer Einsatzposition in einer vertikalen Position gefaltet ist, in einer vertikalen Position senkrecht in Bezug auf das Ende der Windel, das in einer Einsatzposition die Taille bildet, und auf der gleichen Höhe des Lochs (5, 5'), vor diesem oder an eine seitliche Position verschoben aufweist.

20. Anatomischer Artikel nach einem der Ansprüche 14 bis 16, der zwei Systeme (31) zum Öffnen und Verschließen umfasst, die sich in einer Einsatzposition an der Vorderseite der Windel oberhalb der Position des Lochs (5, 5') und unterhalb des oberen Endes des geschichteten Körpers (1) befinden, wobei sich die Systeme (31) in einer schrägen Position in Bezug auf die Länge des gefalteten geschichteten Körpers (1) befinden, um Betätigung zu erleichtern.

21. Anatomischer Artikel nach einem der Ansprüche 17 bis 20, wobei das System (31) zum Öffnen und Verschließen eine längliche Öffnung in dem geschichteten Körper (1) mit einer Klebelasche, einer Klettverschluss-Lasche oder einer lösbaren Verbindungseinrichtung ist, wobei es, wenn es geschlossen ist, die Öffnung abdeckt und sie abdichtet, und wobei die Lasche eine absorbierende Schicht enthält, die die gesamte Innenseite derselben abdeckt und innerhalb des Behälters (2) verbleibt, der Durchlass von Flüssigkeit in den Innenbereich der Lasche verhindert wird, und die absorbierende Schicht einen absorbierenden Kern (33) an ihrer Oberfläche aufweist, der eine Dicke hat, die größer ist als die der zweiten, absorbierenden Schicht (4) des geschichteten Körpers (1) und ausreicht, um die zwei einander zugewandten Flächen der absorbierenden Schicht (4) des gefalteten geschichteten Körpers (1) durch Kontakt zu verbinden.

22. Anatomischer Gegenstand nach einem der vorangehenden Ansprüche, wobei die zweite Schicht (4) an der ersten Schicht (3) an einer der zwei Seiten derselben durch eine lösbare Verbindungseinrichtung haftet.

## Revendications

1. Article absorbant anatomique ayant des propriétés de rétention pour fluides et déchets du corps humain, lequel comprend un seul corps stratifié flexible (1) qui comprend :
- une première couche (3), constituée d'un matériau imperméable,
- une seconde couche (4), constituée d'un matériau absorbant, collée à la première couche (3) sur une de ses deux faces et qui recouvre partiellement ou entièrement la surface de ladite face,
ledit corps stratifié (1) étant configuré pour se plier sur lui-même et délimiter un espace interne fermé, dans la forme dans un récipient (2), de sorte que la couche imperméable (3) constitue la paroi externe en contact avec la peau de l'utilisateur et la couche absorbante (4) constitue la paroi interne du récipient (2) dans la totalité, ou en partie, de son périmètre interne ; et
- une perforation (5, 5') qui passe à travers les deux couches (3, 4) et qui, dans une position d'utilisation, entre en contact avec l'organe urinaire sur la couche externe imperméable (3), de sorte qu'elle permet au fluide d'entrer dans le récipient (2) lorsque le corps stratifié (1) est plié sur lui-même.

2. Article absorbant anatomique ayant des propriétés de rétention pour fluides et déchets du corps humain selon la revendication 1, dans lequel la perforation (5, 5') qui passe à travers les deux couches (3, 4) et qui entre en contact avec l'organe urinaire dans une position d'utilisation est destinée à permettre à l'organe urinaire d'être introduit à partir de la couche externe imperméable (3) jusqu'à la couche interne absorbante (4), de sorte que l'organe urinaire reste à l'intérieur du récipient (2) lorsque le corps (1) est plié sur lui-même.

3. Article anatomique selon l'une quelconque des revendications 1 ou 2, lequel est jetable.

4. Article anatomique selon l'une quelconque des revendications précédentes, dans lequel une partie de la face de la couche imperméable (3) opposée à la face où la couche absorbante (4) est collée présente une troisième couche de couverture (6) collée qui est lisse au toucher, apte à être en contact avec le corps de l'utilisateur, ou dans lequel la face entière de la couche imperméable (3) opposée à la face où la couche absorbante (4) est collée présente une troisième couche (6) collée, laquelle recouvre la totalité de la surface externe de l'article anatomique, à la fois sur sa face externe en contact avec l'environnement et sur sa face interne qui est en contact avec le corps de l'utilisateur dans une position d'utilisation.

5. Article anatomique selon l'une quelconque des revendications précédentes, dans lequel la seconde couche absorbante (4) présente une surface en relief avec un système de canaux (13) dans une forme semblable à une grille à travers laquelle le fluide est distribué à l'intérieur du récipient (2) et qui délimite des noyaux (14) de la couche absorbante (4) où l'absorption desdits fluides est concentrée.

6. Article anatomique selon l'une quelconque des revendications précédentes, dans lequel la face interne de la seconde couche absorbante (4) est recouverte par une couche constituée d'un matériau imperméable (11, 12) qui est identique ou différent de celui de la première couche imperméable (3) du corps stratifié (1) dans deux zones : la zone autour de la perforation (5, 5') et la zone qui est opposée à la perforation (5, 5') lorsque le corps stratifié (1) est plié sur lui-même.

7. Article anatomique selon l'une quelconque des revendications 2 à 6 précédentes, dans lequel le corps stratifié (1) incorpore une canule (10) constituée d'un matériau imperméable élastique et flexible qui passe à travers les couches (3, 4) par la perforation (5, 5'), destinée à recouvrir l'organe urinaire mâle de l'utilisateur.

8. Article anatomique selon la revendication précédente, dans lequel la canule (10) incorpore une bande élastique plate (18) sur sa surface externe avec une longueur suffisante pour entourer ladite canule (10), avec un système d'ouverture et fermeture (19) à ses deux extrémités.

9. Article anatomique selon l'une quelconque des revendications 7 ou 8, dans lequel la canule (10) présente une couche constituée d'un matériau absorbant (15) sur sa face interne, apte à être en contact avec l'utilisateur, dans la forme de bandes longitudinales le long de l'axe de la canule (10) séparées par des encoches (16) qui réalisent un joint d'expansion afin de permettre l'expansion de la canule (10) lors de l'introduction de l'organe urinaire, sans limiter son élasticité ou flexibilité et sans étrangler l'organe urinaire.

10. Article anatomique selon l'une quelconque des revendications 7 à 9, dans lequel la canule (10) est indépendante du corps stratifié (1) et présente une extension plate (20) avec deux faces dans son embouchure, perpendiculaire à l'axe longitudinal de ladite canule (10), plus large que son diamètre afin de former un arrêt, évitant à la canule (10) d'être complètement introduite à l'intérieur du récipient (2) ; et dans lequel :
- la face avant de l'extension (20) qui est en contact avec la couche imperméable (3) de l'article présente un moyen de liaison adhésif ou séparable (21) entre les deux pour fixer ladite extension à la zone autour de la perforation (5, 5') qui est recouverte par l'extension (20) ; et
- la face arrière de l'extension (20) apte à entrer en contact avec le corps de l'utilisateur incorpore sur sa surface une couche de couverture absorbante (23) qui est lisse au toucher pour augmenter par-là le confort.

11. Article anatomique selon l'une quelconque des revendications 2 à 10 précédentes, dans lequel la face de la seconde couche absorbante (4), qui constitue la paroi interne du récipient (2) lorsque le corps stratifié (1) est plié sur lui-même incorpore un noyau (25) dans la zone qui est immédiatement sous la perforation (5, 5'), selon la position d'utilisation, dans laquelle l'organe urinaire est supporté, ledit noyau (25) étant formé par une augmentation dans l'épaisseur de la couche absorbante (4) dans la zone située sous la perforation (5, 5') ou par un élément indépendant, d'un matériau absorbant ou d'un matériau isolant, qui est collé à la couche absorbante (4) dans cette zone, dans la forme d'une saillie ou protubérance ; ledit noyau (25) comprenant une sangle de sécurité élastique (26) dans une direction transversale par rapport à la position longitudinale de l'organe sur le noyau (25), qui présente un système d'ouverture et fermeture (27) sur chacun de ses deux côtés pour rester reliée aux deux côtés du noyau (25) et pour fixer l'organe.

12. Article anatomique selon la revendication précédente, dans lequel le noyau (25) est constitué d'un matériau absorbant, ledit noyau (25) inclut un film de revêtement imperméable (28) sur sa surface qui entre en contact avec l'organe urinaire afin d'éviter l'accumulation d'humidité dans ladite zone et permettre l'absorption sur les deux côtés du noyau (25) qui ne sont pas revêtus par le film imperméable (28).

13. Article anatomique selon l'une quelconque des revendications 11 ou 12, dans lequel le noyau (25) présente sur sa surface une structure de séparation rigide ou flexible (29) qui fait saillie au-dessus de l'organe urinaire apte à rester sur ledit noyau (25), destinée à assurer un creusement dans la zone du récipient (2) autour de la perforation (5, 5') et éviter un compactage, et qui présente une zone centrale interne creuse (30), dans laquelle ledit organe est logé, fournissant une barrière et un support à celui-ci.

14. Article anatomique selon l'une quelconque des revendications précédentes, lequel est une couche absorbante dans laquelle le corps stratifié flexible (1) est déplié lorsque fabriqué et présente des zones de pliage transversale et longitudinale, étant configurées pour se plier sur elles-mêmes vers l'avant dans la forme d'une enveloppe dans une position d'utilisation lorsque placées sur le corps de l'utilisateur, joignant les extrémités du corps stratifié (1) au moyen d'un système d'ouverture et fermeture (9c) qui leur permet d'être scellées et délimitant par-là le récipient interne (2), et dans lequel les côtés (7a, 7b) du corps stratifié (1) présentent des bandes adhésives ou moyens de liaison séparables (9a, 9b) le long de leur longueur entière afin de sceller le récipient (2) une fois pliés.

15. Article anatomique selon la revendication précédente, dans lequel le corps stratifié (1) présente une configuration allongée avec une longueur telle que, une fois qu'il est plié et fermé vers l'avant pour former le récipient (2), sa partie inférieure selon la position d'utilisation est pliée entre les cuisses de l'utilisateur vers leur arrière, entourant leur corps.

16. Article anatomique selon l'une quelconque des revendications 1 à 13, lequel est une couche absorbante dans lequel le corps stratifié flexible (1) est plié sur lui-même lorsque fabriqué, étant relié à ses extrémités et les côtés scellés formant le récipient (2).

17. Article anatomique selon l'une quelconque des revendications 14 à 16, lequel présente au moins un système d'ouverture et fermeture (31) du récipient (2), ledit système d'ouverture et fermeture (31) étant disposé dans une zone du corps stratifié (1) disposée au-dessus de la perforation (5, 5') qui passe à travers les couches (3, 4).

18. Article anatomique selon la revendication précédente, dans lequel le système d'ouverture et fermeture (31) est disposé dans la région centrale de l'extrémité supérieure de la couche, à la hauteur de la taille de l'utilisateur selon la position d'utilisation, ledit système étant accompagné sur les deux côtés par le corps stratifié (1) ; ou à tout autre point de la partie du corps stratifié (1) qui fait face à la perforation (5, 5') lorsque plié dans une position d'utilisation, entre la hauteur de la perforation (5, 5') et de ladite extrémité supérieure de la couche.

19. Article anatomique selon l'une quelconque des revendications 14 à 16, lequel présente un système d'ouverture et fermeture (31) pour le récipient (2) situé dans la zone du corps stratifié (1) qui fait face à la partie où la perforation (5, 5') est logée lorsqu'il est plié dans une position d'utilisation, dans une position verticale perpendiculaire à l'extrémité de la couche qui forme la taille dans une position d'utilisation et à la même hauteur de la perforation (5, 5'), devant celle-ci ou déplacée vers une position latérale.

20. Article anatomique selon l'une quelconque des revendications 14 à 16, qui comprend deux systèmes d'ouverture et fermeture (31) disposés sur la face avant de la couche, au-dessus de la position de la perforation (5, 5') et en dessous de l'extrémité supérieure du corps stratifié (1) dans une position d'utilisation, lesdits systèmes (31) étant dans une position oblique par rapport à la longueur du corps stratifié plié (1) pour faciliter la manipulation.

21. Article anatomique selon l'une quelconque des revendications 17 à 20, dans lequel le système d'ouverture et fermeture (31) est une ouverture allongée dans le corps stratifié (1) avec une patte adhésive, une patte de type Velcro ou un moyen de liaison séparable, de sorte qu'une fois qu'il est fermé, il recouvre ladite ouverture, la scellant, et dans lequel ladite patte incorpore une couche absorbante qui recouvre sa face interne entière et reste à l'intérieur du récipient (2), évitant le passage de liquide dans la zone interne de la patte, et ladite couche absorbante incorpore un noyau absorbant (33) sur sa surface qui présente une épaisseur supérieure à celle de la seconde couche absorbante (4) du corps stratifié (1) et suffisante pour relier par contact les deux faces opposées de la couche absorbante (4) du corps stratifié plié (1).

22. Article anatomique selon l'une quelconque des revendications précédentes, dans lequel la seconde couche (4) est collée à la première couche (3) sur une de ses deux faces par un moyen de liaison séparable.
